# EUROPEAN PATENT SPECIFICATION

(11) **EP 2 942 348 B1**
(45) Date of publication and mention of the grant of the patent: **25.10.2017**
(21) Application number: 14167291.5
(22) Date of filing: 07.05.2014
(51) Int. Cl.: C07D 263/44, C08G 69/10, A61K 8/23, A61K 8/89, A61K 8/90, A61K 31/795, C07K 1/06, C07K 7/08, A61K 9/00, A61K 9/50, A61K 9/51, C07C 323/58, C07C 381/04, C08G 69/40, C08G 73/02

(54) **Thiol-protected amino acid derivatives and uses thereof**
Thiol-geschützte Aminosäurederivate und ihre Verwendungen
Dérivés d'acides aminés protégés par du thiol et leurs utilisations

(43) Date of publication of application: 11.11.2015
(73) Proprietor: Johannes Gutenberg-Universität Mainz, 55122 Mainz (DE)
(72) Inventor: BARZ, Matthias, 67065 Ludwigshafen (DE); HUESMANN, David, 55127 Mainz (DE); REUTER, Thomas, 55118 Mainz (DE)
(74) Representative: Reitstötter Kinzebach

(56) References cited:
- EP-A1- 1 230 934
- EP-A1- 2 599 834
- US-A1- 2005 276 783
- TERANCE W. HART: "Some observations concerning the S-nitroso and S-phenylsulphonyl derivatives of L-cysteine and glutathione", TETRAHEDRON LETTERS, vol. 26, no. 16, 1 January 1985 (1985-01-01), pages 2013-2016, XP055134958, ISSN: 0040-4039, DOI: 10.1016/S0040-4039(00)98368-0
- TERANCE W. HART ET AL: "Triolsulphonate derivatives of amino acids", TETRAHEDRON LETTERS, vol. 26, no. 32, 1 January 1985 (1985-01-01), pages 3879-3882, XP055134960, ISSN: 0040-4039, DOI: 10.1016/S0040-4039(00)89276-X
- JAMES P. WEIDNER ET AL: "Mixed thiolsulfonates and sulfonamides from polyfunctional mercaptans using trifluoromethyl thiosulfonates", JOURNAL OF MEDICINAL CHEMISTRY, vol. 15, no. 5, 1 May 1972 (1972-05-01), pages 564-567, XP055134961, ISSN: 0022-2623, DOI: 10.1021/jm00275a037
- M. OTTNAD ET AL: "Circulardichroismus des disulfidchromophors in S-alkylthio-L-cysteinen und L-glutathion", TETRAHEDRON, vol. 31, no. 9, 1 January 1975 (1975-01-01), pages 1155-1161, XP055134962, ISSN: 0040-4020, DOI: 10.1016/0040-4020(75)85050-2
- L. J. SCHAAD ET AL: "Linear regression analysis of inhibitory potency of organic disulfides against Histoplasma capsulatum", JOURNAL OF MEDICINAL CHEMISTRY, vol. 18, no. 4, 1 April 1975 (1975-04-01), pages 344-351, XP055134963, ISSN: 0022-2623, DOI: 10.1021/jm00238a004
- ILDA MCVEIGH ET AL: "Biologically oriented organic sulfur chemistry. 10. Inhibitory effects of certain organic sulflur compounds on Histoplasma capsulatum", JOURNAL OF MEDICINAL CHEMISTRY, vol. 15, no. 4, 1 April 1972 (1972-04-01), pages 431-433, XP055134964, ISSN: 0022-2623, DOI: 10.1021/jm00274a033
- FIELD ET AL: "Biologically orientated", THE JOURNAL OF ORGANIC CHEMISTRY, AMERICAN CHEMICAL SOCIETY, US, vol. 36, no. 2, 1 January 1971 (1971-01-01), pages 309-313, XP002300617, ISSN: 0022-3263, DOI: 10.1021/JO00801A014
- HAO ZHANG ET AL: "A Kinetic Approach to Characterize the Electrostatic Environments of Thiol Groups in Proteins", BIOORGANIC CHEMISTRY, vol. 26, no. 6, 1 December 1998 (1998-12-01), pages 356-364, XP055134975, ISSN: 0045-2068, DOI: 10.1006/bioo.1998.1112
- FEDERICO MARIA RUBINO ET AL: "Characterization of the disulfides of bio-thiols by electrospray ionization and triple-quadrupole tandem mass spectrometry", JOURNAL OF MASS SPECTROMETRY, vol. 39, no. 12, 1 December 2004 (2004-12-01), pages 1408-1416, XP055134977, ISSN: 1076-5174, DOI: 10.1002/jms.745
- XU J ET AL: "Surface chemistry and photophysical properties of a diacetylene-peptide derivative capped quantum dots Langmuir monolayer", COLLOIDS AND SURFACES. B, BIOINTERFACES, ELSEVIER, AMSTERDAM, NL, vol. 70, no. 2, 1 May 2009 (2009-05-01), pages 163-168, XP026012071, ISSN: 0927-7765, DOI: 10.1016/J.COLSURFB.2008.11.022 [retrieved on 2008-11-30]
- ROBERT L. SHERMAN ET AL: "Semiconductor Nanoparticle/Polystyrene Latex Composite Materials", LANGMUIR, vol. 21, no. 11, 1 May 2005 (2005-05-01), pages 5218-5222, XP055135030, ISSN: 0743-7463, DOI: 10.1021/la0468139
- NEIL C. POMROY ET AL: "Conjugation of Polyethylene Glycol via a Disulfide Bond Confers Water Solubility upon a Peptide Model of a Protein Transmembrane Segment", ANALYTICAL BIOCHEMISTRY, vol. 275, no. 2, 10 June 1999 (1999-06-10) , pages 224-230, XP055135039, ISSN: 0003-2697, DOI: 10.1006/abio.1999.4315
- KIMPLE ET AL: "The RGS protein inhibitor CCG-4986 is a covalent modifier of the RGS4 Galpha-interaction face", BIOCHIMICA ET BIOPHYSICA ACTA (BBA) - PROTEINS & PROTEOMICS, ELSEVIER, NETHERLANDS, vol. 1774, no. 9, 31 August 2007 (2007-08-31), pages 1213-1220, XP022226859, ISSN: 1570-9639, DOI: 10.1016/J.BBAPAP.2007.06.002
- T. LANG ET AL: "Evidence for Cysteine Persulfide as Reaction Product of L-Cyst(e)ine C-S-Lyase (C-DES) fromSynechocystis: ANALYSES USING CYSTINE ANALOGUES AND RECOMBINANT C-DES", JOURNAL OF BIOLOGICAL CHEMISTRY, vol. 274, no. 1, 1 January 1999 (1999-01-01), pages 189-195, XP055135128, ISSN: 0021-9258, DOI: 10.1074/jbc.274.1.189
- E M Cordon ET AL: "Synthesis and Chemistry of Cephalosporin sulfonate esters", Tetrahedron Letters No. Printed inGreat Britain, 12 December 1977 (1977-12-12), pages 1359-1362, XP055135138, Retrieved from the Internet: URL:http://www.sciencedirect.com/science/a rticle/pii/S0040403901930436/pdf?md5=06bd7 399962967b1b7afa62df145b51d&pid=1-s2.0-S00 40403901930436-main.pdf [retrieved on 2014-08-18]
- JOCHEN KRÜGER ET AL: "Manufacturing and PEGylation of a Dual-Acting Peptide for Diabetes", EUROPEAN JOURNAL OF ORGANIC CHEMISTRY, vol. 2008, no. 35, 1 December 2008 (2008-12-01), pages 5936-5945, XP055135192, ISSN: 1434-193X, DOI: 10.1002/ejoc.200800722
- ANDREI LEONOV ET AL: "Convenient Synthesis of Multifunctional EDTA-Based Chiral Metal Chelates Substituted with anS-Mesylcysteine", CHEMISTRY - A EUROPEAN JOURNAL, vol. 11, no. 11, 20 May 2005 (2005-05-20), pages 3342-3348, XP055135193, ISSN: 0947-6539, DOI: 10.1002/chem.200400907
- I. PHOCAS ET AL: "New methods in peptide synthesis. Part IV. N ? S transfer of N-o-nitrophenylsulphenyl groups in cysteine peptides", JOURNAL OF THE CHEMICAL SOCIETY C: ORGANIC, 1 January 1967 (1967-01-01), page 1506, XP055135197, ISSN: 0022-4952, DOI: 10.1039/j39670001506
- Toshihiko Ozawa ET AL: "A kinetic study of the thiol-disulfide exchange reaction between aminothiols and 5,5'-dithiobis(2-nitrobenzoic acid)", CHEMICAL & PHARMACEUTICAL BULLETIN, 1 January 1981 (1981-01-01), pages 1101-1105, XP055135206, Tokyo DOI: 10.1248/cpb.29.1101 Retrieved from the Internet: URL:http://search.proquest.com/docview/145 9941983 [retrieved on 2014-08-19]
- ANNETTE RÖSLER ET AL: "Advanced drug delivery devices via self-assembly of amphiphilic block copolymers", ADVANCED DRUG DELIVERY REVIEWS, vol. 64, 1 December 2012 (2012-12-01), pages 270-279, XP055134998, ISSN: 0169-409X, DOI: 10.1016/j.addr.2012.09.026

## Description

The present invention relates to thiol-protected amino acid derivatives, peptides and block-copolymers and methods for preparing same. The present invention also relates to core-shell particles comprising such thiol-protected block-copolymers and compositions thereof.

### BACKGROUND OF THE INVENTION

Block copolymers are known in research and industry for several decades and have been used for medical applications mainly as excipients in drug formulations and coatings of colloidal particles. For example, block copolymer-drug composites, wherein the copolymer comprises a water-soluble polyethylene oxide block as well as a polypeptide block, are described in EP1514560 and EP2077293.

Amphiphilic block copolymers comprising a water-soluble and a water-insoluble block can form core-shell particles or micelles which are described to be useful as labeling materials or for the encapsulation of therapeutics (cf. EP1792927; EP1398635; Christian et al., Eur J Pharm Biopharm 2009, 71(3):463-474; Hanson etal., Macromolecules 2010, 43(15):6268-6269).

If such core-shell particles are to be used for transporting cargo molecules such as drugs or labelling agents, the particles should be sufficiently stable so as to avoid an untimely release of the cargo before or after administration. At the same time, the particles should readily release the cargo at the target location and be degraded. Thus, a desirable property of core-shell particles is the capability for a site specific release of the cargo (usually by disintegration of the particle structure) such as after uptake by a cell in a specific intracellular compartment, combined with stability in a liquid media used in pharmaceutical formulations (such as saline) or in bodily fluids (such as blood or lymph). It was an object of the present invention to provide core-shell particles which meet these requirements.

In case of block copolymers as previously described, a covalent coupling of cargo molecules to the block copolymers or a cross-linking of block copolymers within core-shell particles to increase particles stability requires complicated multi-step procedures including deprotection and activation steps (cf., e.g., EP2258397). Moreover, the thus prepared particles may contain residual agents from such cross-linking or cargo coupling process, e.g., residual cross-linking agent and initiator, which are potentially toxic to animals and humans in *in vivo* applications and may adversely affect the stability of the cargo molecule.

It was therefore an object of the present invention to provide polymer-based core-shell particles which, without the need for harsh reaction conditions and while maintaining the core-shell structure, can be stabilized by internal cross-linking, and/or can be chemically modified so as to attach cargo molecules and/or adjust the hydrophobicity of polymer. (As described below, such cross-linking or modification is effected according to the present invention by the formation of disulfide bonds which are known to be cleaved intracellularly.)

It was a further object of the present invention to provide amino acid derivatives and block copolymers which can be used for preparing such core-shell particles.

A common method for polypeptide synthesis is the stepwise addition of amino acids to the N-terminus of the growing polypeptide chain. An alternative method is the ring-opening polymerization (ROP) of amino acid N-carboxyanhydrides (amino acid NCAs) (cf., e.g., Hadjichristidis et al., Chem. Re. 2009, 209:5528-5578). Apart from the group forming the peptide bond that couples the amino acid to the growing polypeptide chain (usually the carboxy group of the amino acid), amino acids carry further reactive groups (e.g., amino, carboxy, hydroxyl and thiol groups) which can interact with free termini or side chain groups during polypeptide synthesis and thus reduce yield and purity of the polypeptide product. The same applies to amino acid NCAs. These further reactive groups are therefore usually blocked by the addition of chemical groups (protecting groups) which protect the further reactive groups from non-specific reactions. Such non-specific reactions include the formation of undesired disulfide bridges, thioesters or thioethers involving thiol-containing amino acids such as cysteine and homocysteine. Commonly used thiol-protecting groups include acetamidomethyl, tert-butyl and triphenylmethyl groups. However, thiol-protecting groups which are stable under the conditions of polypeptide synthesis are often not sufficiently reactive against free thiol groups and thus prevent the reaction of the thus protected thiol group with other thiols. Such thiol-protecting groups therefore have to be removed prior to further modifications at the thiol group.

It was therefore a further object of the present invention to provide thiol-protecting groups and corresponding thiol-protected amino acid derivatives which are stable under the conditions of polypeptide synthesis (e.g. by ROP) and synthesis of amino acid NCAs from amino acids, and at the same time allow the protected thiol groups to be further modified (in particular by forming disulfide bonds to free thiols) without prior deprotection.

Amino acid derivatives comprising a modified thiol group are described in Hart (Tetrahedron Letters 1985, 26(16), 2013-2016), Hart et al. (Tetrahedron Letters 1985, 26(32), 3879-3882), Weidner et al. (J Med Chem 1972, 15(5), 564-567), Ottnad et al. (Tetrahedron 1975, 31, 1155-1161), Schaad et al. (J Med Chem 1975, 18(4), 344-351), McVeigh et al. (J Med Chem 1972, 15(4), 431-433), Field et al. (J Org Chem 1971, 36(2), 309-313), Zhang et al. (Bioorg Chem 1998, 26, 356-364), Rubino et al. (J Mass Spectrom 2004, 39, 1408-1416), Kimple et al. (Biochim Biophys Acta 2007, 1774, 1213-1220), Lang et al. (J Biol Chem 1999, 274(1), 189-195), Gordon et al. (Tetrahedron Lett 1977, 16, 1359-1362), Krüger et al. (Eur J Org Chem 2008, 5936-5945), Loenov et al. (Chem Eur J 2005, 11, 3342-3348), Phocas et al. (J Chem Soc 1967, 1506-1509) and Ozawa et al. (Chem Pharm Bull 1981, 29(4), 1101-1105). Pomroy et al. (Anal Biochem 1999, 275, 224-230) describes a polymer comprising a cysteine-derived monomeric unit haing a modified thiol-group. Polymer comprising thiol-groups and the crosslinking of said groups after deprotection or reduction, respectively, is described in EP 2 599 834 A1, US 2005/0276783 A1 and EP 1 230 934 A1.

### SUMMARY OF THE INVENTION

The present invention provides a thiol-protected amino acid derivative of formula (I) or formula (II) 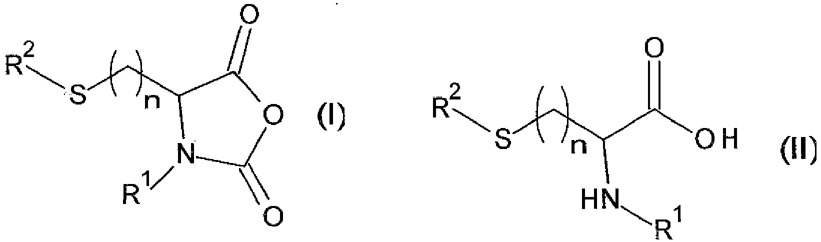 wherein
n is 1 or 2;
R¹ is H, C₁-C₄-alkyl, C₂-C₄-alkenyl or C₂-C₄-alkynyl; and
R² is a group of formula (A) or formula (B) 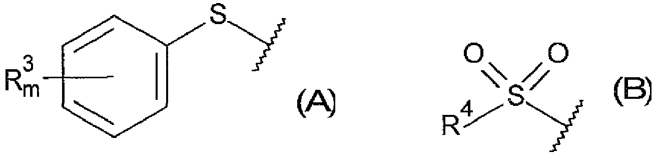 wherein
   m is 1, 2, 3, 4 or 5;
   each R³ is independently selected from halogen, cyano and C₁-C₄-alkanoyl; and R⁴ is selected from R^{a}-C₁-C₁₆-alkyl, alkyl selected from ethyl, n-propyl, isopropyl, n-butyl, 1-methylpropyl (sec-butyl), 2-methylpropyl (isobutyl) and 1,1-dimethylethyl (tert-butyl), n-pentyl, 1-methylbutyl, 2-methylbutyl, 3-methylbutyl (isopentyl), 2,2-dimethylpropyl, 1-ethylpropyl, 1,1-dimethylpropyl, 1,2-dimethylpropyl, n-hexyl, 1-methylpentyl, 2-methylpentyl, 3-methylpentyl, 4-methylpentyl (isohexyl), 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, n-octyl, 2-methylheptyl, 1-ethylhexyl, 2-ethylhexyl, 1,2-dimethylhexyl, 1-propylpentyl, 2-propylpentyl, C₂-C₁₆-alkenyl and C₂-C₁₆-alkynyl, wherein the C₁-C₁₆-alkyl, ethyl, n-propyl, isopropyl, n-butyl, 1-methylpropyl (sec-butyl), 2-methylpropyl (isobutyl) and 1,1-dimethylethyl (tert-butyl), n-pentyl, 1-methylbutyl, 2-methylbutyl, 3-methylbutyl (isopentyl), 2,2-dimethylpropyl, 1-ethylpropyl, 1,1-dimethylpropyl, 1,2-dimethylpropyl, n-hexyl, 1-methylpentyl, 2-methylpentyl, 3-methylpentyl, 4-methylpentyl (isohexyl), 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, n-octyl, 2-methylheptyl, 1-ethylhexyl, 2-ethylhexyl, 1,2-dimethylhexyl, 1-propylpentyl, 2-propylpentyl, C₂-C₁₆-alkenyl or C₂-C₁₆-alkynyl are unsubstituted or carry 1, 2 or 3 substituents R^{b};
   R^{b} is independently selected from halogeno, cyano, nitro, hydroxyl and thiol;
   R^{a} is selected from:
      (i) phenyl,
      (ii) 5- or 6-membered heteroaromatic monocyclic radicals having 1, 2, 3 or 4 heteroatoms as ring members which are independently selected from O, S and N,
      (iii) 8- to 10-membered heteroaromatic bicarbocyclic radicals having 1, 2, 3 or 4 heteroatoms as ring members which are independently selected from O, S and N, and
      (iv) 8- to 10-membered aromatic bicarbocyclic radicals,
      wherein said radicals (i) to (iv) are unsubstituted or carry 1, 2, 3 or 4 substituents R^{c}, and
      R^{c} is independently selected from halogeno, cyano, nitro, hydroxyl, C₁-C₄-alkyl, C₁-C₄-alkoxy, C₂-C₄-alkenyl, C₂-C₄-alkynyl and C₁-C₄-alkanoyl.

The invention further provides a method for preparing a peptide comprising at least one amino acid residue that carries an, optionally protected, thiol-group; the method comprising reacting a thiol-protected amino acid derivative as described herein with an amino acid or with an amino acid residue of a precursor peptide under coupling conditions, thus obtaining a peptide comprising at least one thiol-protected amino acid residue, wherein the thiol-protected amino acid derivative is a compound of formula (I) or formula (II) 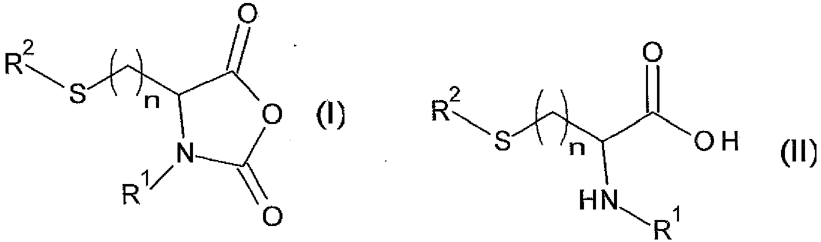 wherein
n is 1 or 2;
R¹ is H, C₁-C₄-alkyl, C₂-C₄-alkenyl or C₂-C₄-alkynyl; and
R² is a group of formula (A) or formula (B) 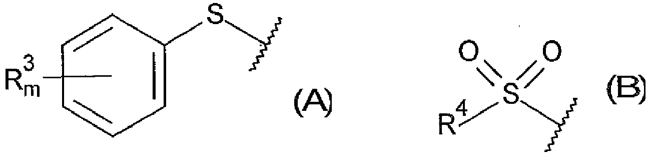 wherein
   m is 1, 2, 3, 4 or 5, and each R³ is independently selected from halogen, nitro, cyano and C₁-C₄-alkanoyl;
      preferably m is 1, 2, 3, 4 or 5, and each R³ is independently selected from fluoro, chloro and bromo;
      more preferably m is 5, and R³ is chloro, orm is 1, 2 or 3, and R³ is fluoro;
   R⁴ is selected from R^{a}-C₁-C₁₆-alkyl, R^{a}, C₁-C₁₆-alkyl, C₂-C₁₆-alkenyl and C₂-C₁₆-alkynyl, wherein the C₁-C₁₆-alkyl, C₂-C₁₆-alkenyl or C₂-C₁₆-alkynyl are unsubstituted or carry 1, 2 or 3 substituents R^{b};
      preferably R⁴ is selected from C₁-C₁₆-alkyl, R^{a}-C₁-C₁₆-alkyl and R^{a}, or R⁴ is selected from unsubstituted C₂-C₁₆-alkenyl and partly or completely halogenated C₂-C₁₆-alkenyl;
      more preferably R⁴ is selected from ethyl, butyl, hexyl and benzyl, wherein the benzyl is unsubstituted or carries 1, 2, 3 or 4 substituents R^{c};
   R^{b} is independently selected from halogeno, cyano, nitro, hydroxyl and thiol;
   R^{a} is selected from:
      (i) phenyl,
      (ii) 5- or 6-membered heteroaromatic monocyclic radicals having 1, 2, 3 or 4 heteroatoms as ring members which are independently selected from O, S and N,
      (iii) 8- to 10-membered heteroaromatic bicarbocyclic radicals having 1, 2, 3 or 4 heteroatoms as ring members which are independently selected from O, S and N, and
      (iv) 8- to 10-membered aromatic bicarbocyclic radicals,
      wherein said radicals (i) to (iv) are unsubstituted or carry 1, 2, 3 or 4 substituents R^{c}; and
   R^{c} is independently selected from halogeno, cyano, nitro, hydroxyl, C₁-C₄-alkyl, C₁-C₄-alkoxy, C₂-C₄-alkenyl, C₂-C₄-alkynyl and C₁-C₄-alkanoyl.

The present disclosure also provides a method of intermediate thiol protection comprising:
(a) providing a first thiol compound having at least one functional group other than a thiol group, wherein at least one thiol group of said first thiol compound is protected by a group of formula (A) or formula (B) 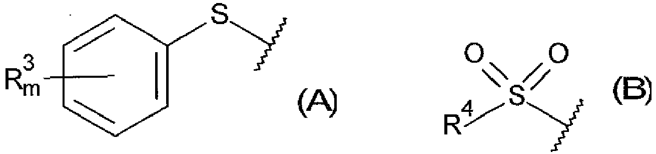 wherein m, R³ and R⁴ are as defined herein,
   so as to form a disulfide with the group of formula (A) or a thiolsulfonate with the group of formula (B);
(b) effecting a chemical reaction on at least one functional group of the thiol-protected compound produced in step (i) under conditions to which the protected thiol group is chemically inert but to which an unprotected thiol group would be chemically reactive; and
(c) subsequently either reacting at least part of the protected thiol groups with the thiol group(s) of a second thiol compound, thus obtaining a disulfide compound, or deprotecting the protected thiol group(s).

The invention also provides a block copolymer comprising a hydrophilic block and a thiol-reactive block, wherein the hydrophilic block is a linear polymer of 10-1000 monomeric units, and the thiol-reactive block is a linear polymer of 1-1000 monomeric units of formula (C) 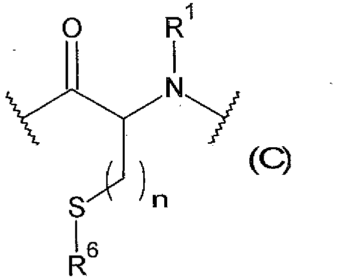 wherein
R⁶ is independently selected from H, a group of formula (A) and a group of formula (B) 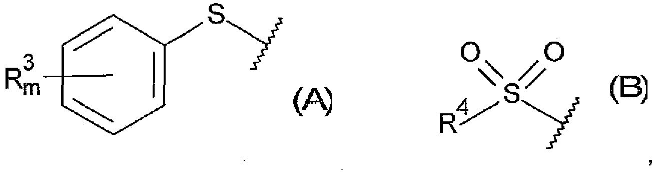 and
n, m, R¹, R³ and R⁴ are as defined herein, wherein in at least one monomeric unit of formula (C) R⁶ is a group of formula (A) or formula (B).

The invention also provides a method for preparing a block copolymer (e.g. a block copolymer as described herein) comprising:
(i) providing a hydrophilic polymer of 10-1000 monomeric units that carries at least one terminal primary or secondary amino group; and
(ii) reacting a thiol-protected amino acid derivative of formula (I) as defined herein with the terminal amino group of the hydrophilic polymer,
thus forming a polymer that carries a terminal primary or secondary amino group.

The invention also provides a core-shell particle comprising
a multitude of block copolymers as described herein, and
a pharmaceutically active agent, a signal generating substance (e.g. a fluorescent dye, enzyme of radio-labelled substance) or a polynucleotide encapsulated therein;
wherein the hydrophilic blocks of the block copolymers are located in the shell region of the particle, and the thiol-reactive blocks of the block copolymers as well as the pharmaceutically active agent, a signal generating substance or polynucleotide are located in the core region of the particle.

The invention also provides a method for stabilizing a core-shell particle comprising a multitude of block copolymers as described herein; the method comprising contacting the core-shell particle with a cross-linker comprising two or more than two thiol groups.

The invention also provides a composition comprising a core-shell particle as described herein and a pharmaceutically acceptable carrier.

### DESCRIPTION OF FIGURES

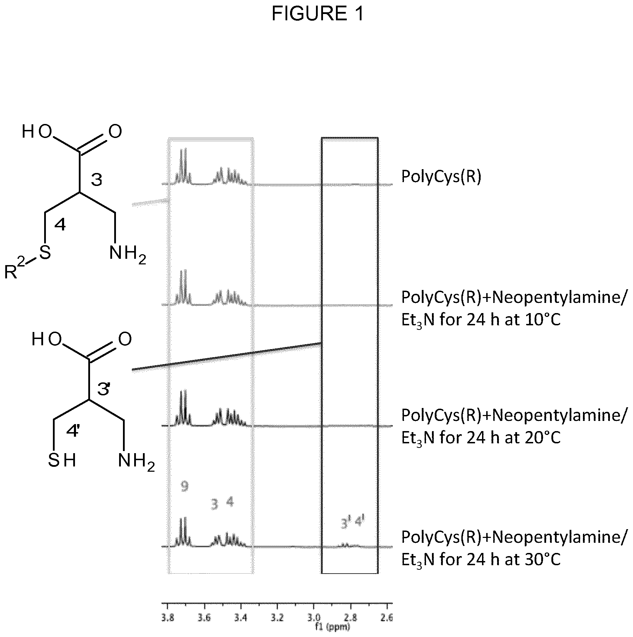

Figure 1 shows NMR analyses of a thiol-protected cysteine of the invention (the thiol-protective group R² in the depicted formula is ethylsulfonyl) (left box) and a polymer thereof ("PolyCys(R)", right box) before and after treatment with neopentylamine for 24 h at 10°C, 20°C or 30°C, respectively. The peaks at positions "3" and "4" are characteristic for the monomer before polymerization. the peaks at position "3' " and "4' " are characteristic for free thiol groups as formed by cleavage of the thiol-protective groups. The Figure illustrates that the polymer of the invention was basically stable against neopentylamine.

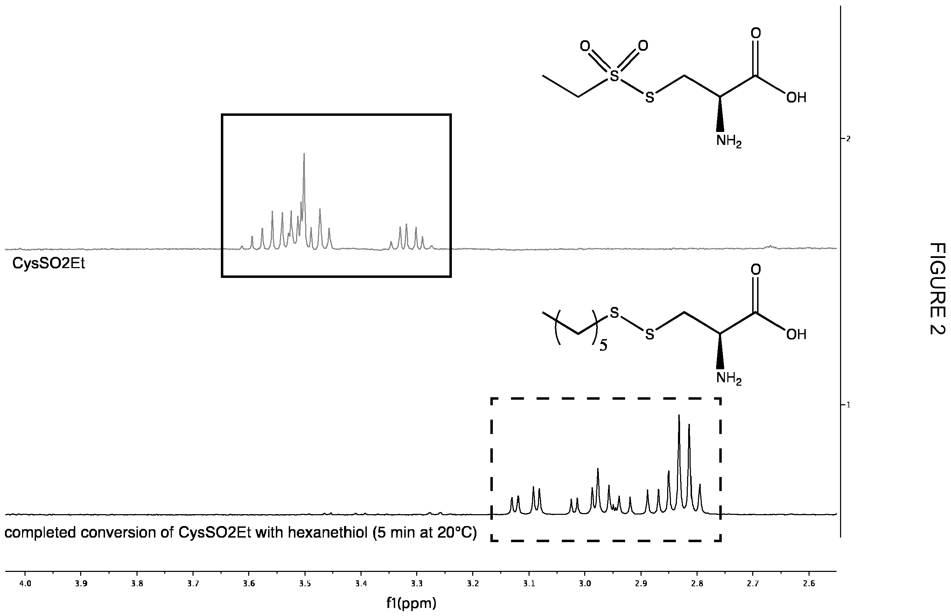

Figure 2 shows NMR analyses of a thiol-protected cysteine of the invention before and after treatment with hexanethiol for 5 min at 20°C. The thiol-protective group is ethylsulfonyl. The Figure illustrates that the thiol-protected cysteine of the invention reacts with hexanethiol yielding quantitatively the disulfide 1-(hexyldisulfanyl)cysteine.

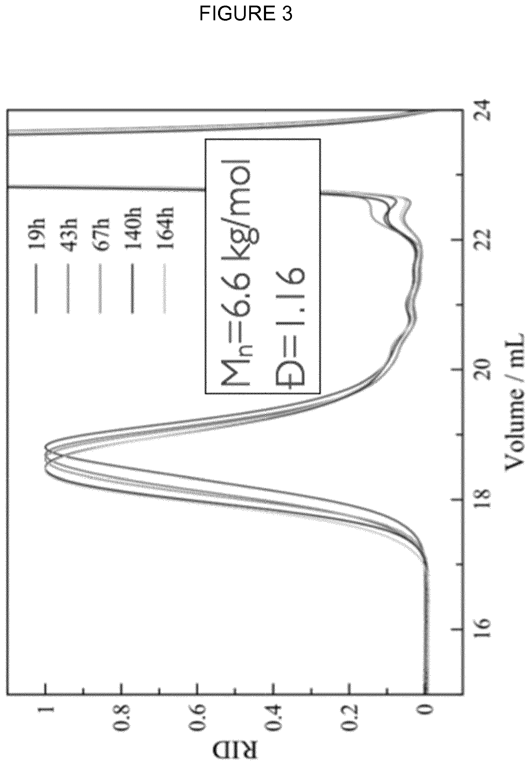

Figure 3 shows an overlay of GPC elugrams of samples taken during the preparation of poly((benzylsulfonylsulfanyl)cysteine) via ROP of the corresponding monomers using neopentylamine. The Figure illustrates the steady chain elongation and the formation of a uniform polymer (peaks having their maximums between -18-19.5 Vol/ml). The number-average molecular weight (Mₙ) of the final polymer was 6,600 g/mol and the dispersity (Ð) was 1.16.

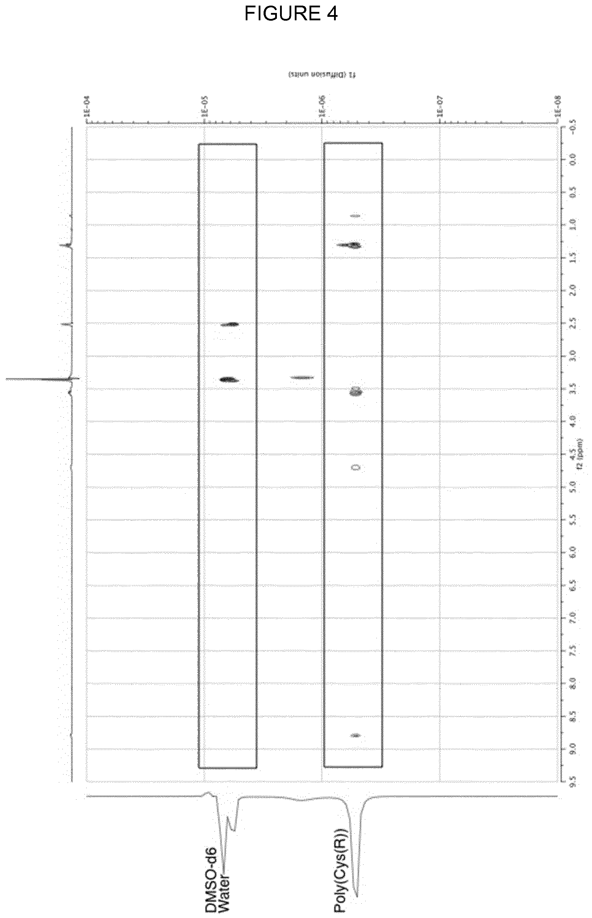

Figure 4 shows a DOSY-NMR spectrum of a thiol-protected poly(cysteine) ("Poly Cys(R)") of the invention obtained by ring opening polymerization of the corresponding monomers using neopentylamine. The thiol-protective groups at the poly(cysteine) are ethylsulfonyl. The Figure shows that there is only one diffusion species besides the solvents (DMSO, water), illustrating that the prepared polymer is uniform.

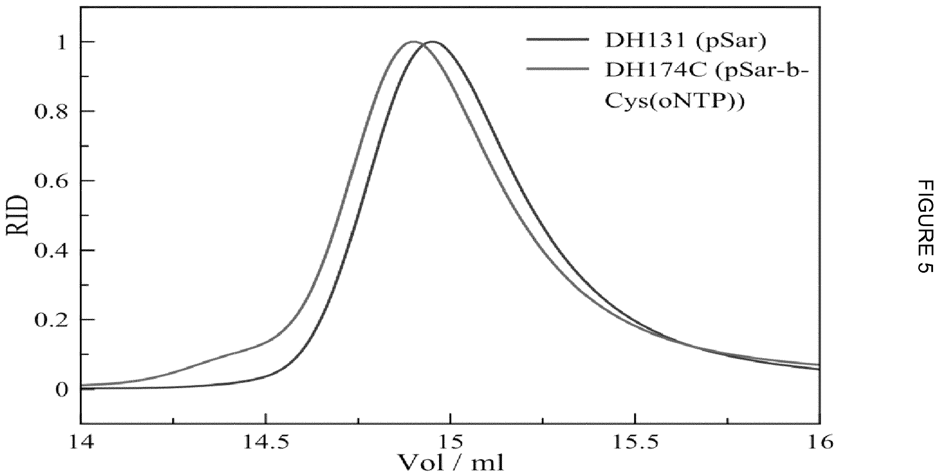

Figure 5 shows an overlay of GPC elugrams of a thiol-protected polysarcosine-block-polycysteine copolymer preparation (left peak) and of a polysarcosine preparation (right peak), each obtained by sequential ring opening polymerization of the corresponding monomers using neopentylamine. The thiol-protective groups at the polycysteine block are benzylsulfonyl. The Figure illustrates the chain elongation by addition of the polycysteine block and the uniformity of the polymer preparations.

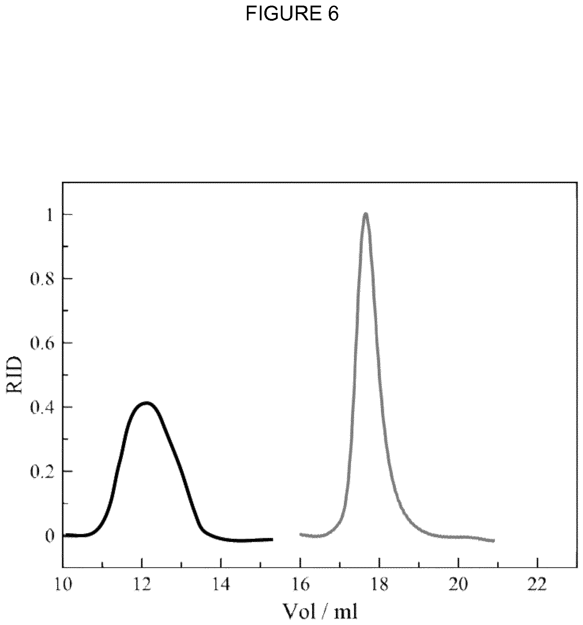

Figure 6 shows an overlay of GPC elugrams of a preparation of thiol-protected polysarcosine(200)-block-polycysteine(20) copolymer (right) and of core-cross-linked micelles (PeptoMicelles) formed from such copolymer (left). The thiol-protective groups at the polycysteine block are benzylsulfonyl. The Figure illustrates the uniformity of the copolymer and cross-linked micelles.

### DETAILED DESCRIPTION OF THE INVENTION

The present invention utilizes the properties of thiol-groups which are protected with particular protecting groups, namely groups of formulae (A) or (B) as described herein. The group of formula (A) together with the thus protected thiol group forms a disulfide group of formula (A') 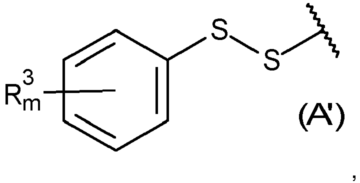 wherein m and R³ are as defined herein. The group of formula (B) together with the thus protected thiol group forms a thiolsulfonate group of formula (B') 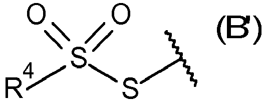 wherein R⁴ is as defined herein.

Unless indicated otherwise, the term "protected thiol groups" is used herein to designate thiol groups protected with groups of formulae (A) or (B) as described herein. Accordingly, as used herein, the term "thiol-protected" refers to a compound or residue (e.g. an amino acid residue) comprising at least one protected thiol group.

Advantageously, the protected thiol groups described herein are relatively stable under acidic conditions as well as under basic conditions and also against different amines, while at the same time they are highly reactive against other thiols, and can therefore be considered activated thiol groups. They can be used for selective linkage with any compound bearing at least one free thiol group. Groups of formulae (A) and (B) can be used in amino acids subjected to the conversion to N-carboxyanhydrides (NCAs) and subsequent ring-opening polymerization. In contrast, the use of other protecting groups for thiols does either not provide sufficiently reactivity or not sufficient stability of the protected thiol groups. For example, thiols protected with groups such as benzylsulfanyl groups are not reactive against free thiol groups and therefore need to be removed prior to further modifications at the thiol group. On the other hand, thiols protected with groups such as 2-pyridylsulfanyl, 4-pyridylsulfanyl or (2-nitro-4-pyridyl)sulfanyl are not sufficiently stable under acidic or basic conditions or the conditions applied in peptide synthesis such as for polymerization of amino acids / amino acyl NCAs or removal of amino-protecting groups (such as Fmoc), and therefore can only be introduced as terminal amino acids of a synthesized peptide.

The phrase "deprotecting" when used with regard to a protected thiol group refers to the removal of the protecting group so as to recover the free thiol group.

The term "amino acid" generally refers to a compound comprising at least one amino group and at least one carboxyl group. The at least one amino group of an "amino acid" may be unsubstituted (-NH₂) or N-monosubstituted (-NHR, wherein R is, for example, R¹ as defined herein).

The term "amino acid residue", as used herein, refers to a monomeric unit of a peptide (such as of a dipeptide, oligopeptide or polypeptide). An "amino acid" may be unsubstituted (i.e. may comprise -NH-) or N-monosubstituted (i.e. may comprise -NR-, wherein R is, for example, R¹ as defined herein).

The term "oligopeptide", as used herein, refers to a peptide of 2 to 9 monomeric units, i.e. amino acid residues. The term "polypeptide", as used herein, refers to a peptide of 10 or more than 10 monomeric units. Peptides such as dipeptides, oligopeptides or polypeptides may be unsubstituted (i.e. comprise only peptide bond of formula -C(O)-NH-) or may be N-monosubstituted (i.e. may comprise at least one peptide bond of formula -C(O)-NR-, wherein R is, for example, R¹ as defined herein).

The term "alkyl" refers to saturated linear or branched hydrocarbon radicals. Accordingly, the term "C₁-C₄-alkyl" refers to saturated linear or branched hydrocarbon radicals having 1, 2, 3 or 4 carbon atoms. Likewise, "C₁-C₈-alkyl" refers to saturated linear or branched hydrocarbon radicals having 1, 2, 3, 4, 5, 6, 7 or 8 carbon atoms. And "C₁-C₁₆-alkyl" refers to saturated linear or branched hydrocarbon radicals having from 1 to 16 carbon atoms. Examples of C₁-C₄-alkyl and C₁-C₆-alkyl include methyl, ethyl, n-propyl, isopropyl, n-butyl, 1-methylpropyl (sec-butyl), 2-methylpropyl (isobutyl) and 1,1-dimethylethyl (tert-butyl). Examples of C₁-C₈-alkyl further include n-pentyl, 1-methylbutyl, 2-methylbutyl, 3-methylbutyl (isopentyl), 2,2-dimethylpropyl, 1-ethylpropyl, 1,1-dimethylpropyl, 1,2-dimethylpropyl, n-hexyl, 1-methylpentyl, 2-methylpentyl, 3-methylpentyl, 4-methylpentyl (isohexyl), 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, n-octyl, 2-methylheptyl, 1-ethylhexyl, 2-ethylhexyl, 1,2-dimethylhexyl, 1-propylpentyl and 2-propylpentyl.

The term "alkoxy" refers to saturated linear or branched hydrocarbon radicals which are bound to the remainder of the molecule via an oxygen atom. Accordingly, the term "C₁-C₄-alkoxy" refers to saturated linear or branched hydrocarbon radicals having 1, 2, 3 or 4 carbon atoms which are bound to the remainder of the molecule via an oxygen atom. Examples of C₁-C₄-alkoxy include methoxy, ethoxy, n-propoxy, isopropoxy, n-butoxy, 1-methylpropoxy (sec-butoxy), 2-methylpropoxy (isobutoxy) and 1,1-dimethylethoxy (tert-butoxy).

The term "alkenyl" refers to unsaturated linear or branched hydrocarbon radicals having one or more than one C-C double bond in any position. Accordingly, the term "C₂-C₄-alkenyl" refers to unsaturated linear or branched hydrocarbon radicals having 2, 3 or 4 carbon atoms and a C-C double bond in any position, preferably at the most terminal position. Likewise, "C₂-C₈-alkenyl" refers to unsaturated linear or branched hydrocarbon radicals having 2, 3, 4, 5 or 6 carbon atoms and at least one C-C double bond in any position, preferably a C-C double bond at the most terminal position. And "C₂-C₁₆-alkenyl" refers to saturated linear or branched hydrocarbon radicals having from 1 to 16 carbon atoms and at least one C-C double bond in any position, preferably a C-C double bond at the most terminal position. Examples of C₂-C₄-alkenyl and C₂-C₈-alkenyl include, but are not limited to, ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl and 2-methyl-2-propenyl. Examples of C₂-C₆-alkenyl further include 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The term "alkynyl" refers to unsaturated linear or branched hydrocarbon radicals having one or more than one C-C triple bond in any position. Accordingly, the term "C₂-C₄-alkynyl" refers to linear or branched hydrocarbon groups having 2, 3 or 4 carbon atoms and a C-C triple bond in any position. Likewise, "C₂-C₁₆-alkynyl" refers to unsaturated linear or branched hydrocarbon radicals having from 2 to 16 carbon atoms and at least one C-C triple bond in any position, preferably a C-C triple bond at the most terminal position. Examples of C₂-C₄-alkynyl and C₂-C₁₆-alkynyl include, but are not limited to, ethynyl, 1-propynyl, 2-propynyl, 1-methylethynyl, 1-butynyl, 2-butynyl, 3-butynyl, 1-methyl-1-propynyl, 2-methyl-1-propynyl, 1-methyl-2-propynyl and 2-methyl-2-propynyl. Examples of C₂-C₁₆-alkynyl further include 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl.

The term "alkanoyl" refers to a carbonyl group that is optionally substituted with a saturated linear or branched hydrocarbon radical. Accordingly, the term "C₁-C₄-alkanoyl" refers to formyl and to saturated linear or branched hydrocarbon radicals having 1, 2 or 3 carbon atoms which are bound to the remainder of the molecule via a carbonyl group so to have a total of 2, 3 or 4 carbon atoms. Examples of C₁-C₄-alkanoyl include formyl, acetyl, propanoyl, 2-methylpropanoyl and butanoyl.

The term "5- or 6-membered heteroaromatic monocyclic radicals" refers to refers to monocyclic aromatic radicals having 5 or 6 ring atoms, wherein 1, 2, 3 or 4, in particular 1, 2 or 3, of the ring atoms are heteroatoms selected from N, S and O, and the remaining ring atoms are carbon atoms, and double bonds between ring members form an aromatic system. 5- or 6-membered heteroaromatic monocyclic radicals may be bound to the remainder of the molecule at a carbon ring atom or at a nitrogen ring atom. Examples of 5- or 6-membered heteroaromatic monocyclic radicals include:
C-bonded, 5-membered aromatic heteromonocyclic rings such as 2-furyl, 3-furyl, 2-thienyl, 3-thienyl, pyrrol-2-yl, pyrrol-3-yl, pyrazol-3-yl, pyrazol-4-yl, isoxazol-3-yl, isoxazol-4-yl, isoxazol-5-yl, isothiazol-3-yl, isothiazol-4-yl, isothiazol-5-yl, imidazol-2-yl, imidazol-4-yl, oxazol-2-yl, oxazol-4-yl, oxazol-5-yl, thiazol-2-yl, thiazol-4-yl, thiazol-5-yl, 1,2,3-oxadiazol-4-yl, 1,2,3-oxadiazol-5-yl, 1,2,4-oxadiazol-3-yl, 1,2,4,-oxadiazol-5-yl, 1,3,4-oxadiazol-2-yl, 1,2,3-thiadiazol-4-yl, 1,2,3-thiadiazol-5-yl, 1,2,4-thiadiazol-3-yl, 1,2,4-thiadiazol-5-yl, 1,3,4-thiadiazol-2-yl, 1,2,3-triazol-4-yl, 1,2,4-triazol-3-yl and tetrazol-5-yl;
C-bonded, 6-membered aromatic heteromonocyclic rings such as 2-pyridyl, 3-pyridyl, 4-pyridyl, pyridazin-3-yl, pyridazin-4-yl, pyrimidin-2-yl, pyrimidin-4-yl, pyrimidin-5-yl, pyrazin-2-yl, 1,3,5-triazin-2-yl, 1,2,4-triazin-3-yl, 1,2,4-triazin-5-yl, 1,2,4-triazin-6-yl and 1,2,4,5-tetrazin-3-yl;
N-bonded, 5-membered aromatic heteromonocyclic rings such as pyrrol-1-yl, pyrazol-1-yl, imidazol-1-yl, 1,2,3-triazol-1-yl, 1,2,4-triazol-1-yl and tetrazol-1-yl; and N-bonded, 6-membered aromatic heteromonocyclic rings such as 1-pyridyl, pyridazin-1-yl, pyrimidin-1-yl, pyrazin-1-yl, 1,3,5-triazin-1-yl, 1,2,4-triazin-1-yl, 1,2,4-triazin-2-yl, 1,2,4-triazin-4-yl and 1,2,4,5-tetrazin-1-yl.

The term "8- to 10-membered heteroaromatic bicarbocyclic radicals" refers to bicarbocyclic aromatic radicals which have 8, 9 or 10 ring atoms, wherein 1, 2, 3 or 4, in particular 1, 2 or 3, of the ring atoms are heteroatoms selected from N, S and O, and the remaining ring atoms are carbon atoms, and which include an aromatic system such as a fused benzene ring or fused 5- or 6-membered heteroaromatic monocyclic radical. 8- to 10-membered heteroaromatic bicarbocyclic radicals may be bound to the remainder of the molecule at a carbon ring atom or at a nitrogen ring atom. Examples of 8- to 10-membered heteroaromatic bicarbocyclic radicals include indolyl, isoindolyl, indolinyl, isoindolinyl, indazolyl, quinolyl, isoquinolyl, dihydroquinolyl, dihydroisoquinolyl, chromenyl, chromanyl, quinazolinyl, dihydrobenzofuryl, dihydrobenzodioxinyl, cinnolinyl, benzofuryl, benzothienyl, benzo[b]thiazolyl, benzoxazolyl, benzthiazolyl and benzimidazolyl.

The term "8- to 10-membered aromatic bicarbocyclic radicals" refers to bicarbocyclic aromatic radicals having 8, 9 or 10 carbon atoms as ring members and include an aromatic system such as a fused benzene ring. Examples of 8- to 10-membered aromatic bicarbocyclic radicals include inden-1-yl, inden-2-yl, inden-4-yl, inden-6-yl, tetrahydro-1-naphthyl, tetrahydro-2-naphthyl, tetrahydro-5-naphthyl, tetrahydro-6-naphthyl, and in particular naphthyl, such as 1-naphthyl and 2-naphthyl.

In the compounds and methods described herein, where the thiol-protecting group is a group of formula (A) 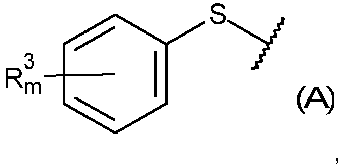 m can be 1, 2, 3, 4 or 5; and each R³ can independently be selected from halogen, nitro, cyano and C₁-C₄-alkanoyl, in particular from halogen, cyano and C₁-C₄-alkanoyl, and more specifically from halogen. Specifically, the group of formula (A) can be selected from groups of formulae (A-1), (A-2), (A-3), (A-4) and (A-5) 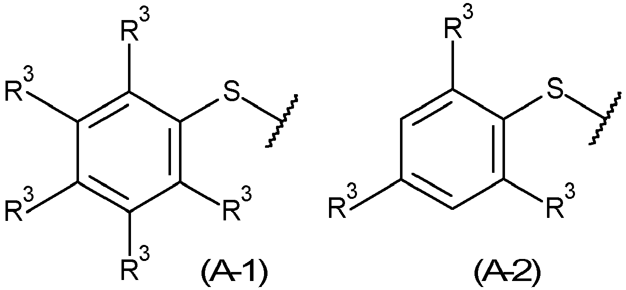 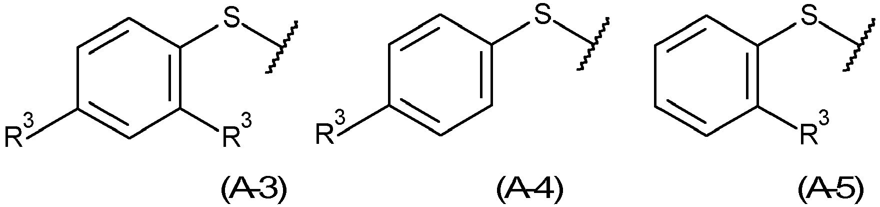 wherein R³ is as defined herein. According to a particular group of embodiments, each R³ is independently selected from fluoro, chloro and bromo. According to a further particular group of embodiments, m is 5; and R³ is chloro. According to a further particular group of embodiments, m is 1, 2 or 3; and R³ is fluoro. In particular in the method for preparing a peptide comprising at least one (optionally protected) thiol-group and in the method of intermediate thiol protection described herein, wherein the thiol-protecting group is a group of formula (A), it is preferred that said group of formula (A) is as defined herein, provided that it is not (2-nitrophenyl)sulfanyl, (4-nitrophenyl)sulfanyl or (2,4-dinitrophenyl)sulfanyl.

According to one aspect, the invention provides thiol-protected amino acid N-carboxyanhydrides of formula (I) 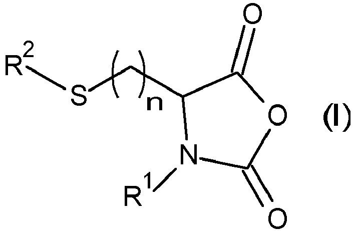 wherein
n is 1 or 2;
R¹ is H, C₁-C₄-alkyl, C₂-C₄-alkenyl or C₂-C₄-alkynyl; and
R² is a group of formula (A) or formula (B) as described herein.

According to an alternative aspect, the invention provides thiol-protected amino acid derivatives of formula (II) 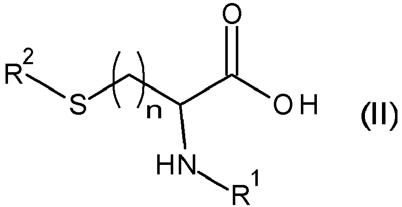 wherein
n is 1 or 2;
R¹ is H, C₁-C₄-alkyl, C₂-C₄-alkenyl or C₂-C₄-alkynyl; and
R² is a group of formula (A) or formula (B) as described herein.

In one group of embodiments of compounds having formula (I) and a corresponding group of embodiments of compound having formula (II), n is 1; R¹ is H, C₁-C₄-alkyl, C₂-C₄-alkenyl or C₂-C₄-alkynyl; and R² is a group of formula (A) or formula (B) as described herein. In a preferred particular group of embodiments, R¹ is H, methyl, ethyl, propargyl or but-3-enyl.

In an alternative group of embodiments of compounds having formula (I) and a corresponding group of embodiments of compound having formula (II), n is 2; R¹ is H, C₁-C₄-alkyl, C₂-C₄-alkenyl or C₂-C₄-alkynyl; and R² is a group of formula (A) or formula (B) as described herein. In a preferred particular group of embodiments, R¹ is H, methyl, ethyl, propargyl or but-3-enyl.

The compounds of formula (I) are N-carboxyanhydrides (NCAs) of the corresponding compounds of formula (II). The expression "corresponding" when used with respect to compounds of formulae (I) and (II) indicates that these compounds have the same integer n and same residues R¹ and R².

Compounds of formula (I) can be prepared using methods for preparing amino acid NCAs known the art, for example by methods as described by the review of Kricheldorf (Angew. Chem. Int. Ed. 2006, 45: 5752-5784) and the literature cited therein. Expediently, compounds of formula (I) as described herein can be prepared by cyclization of corresponding thiol-protected amino acid derivatives of formula (II).

Amino acid derivatives of the present invention having formula (II-A) 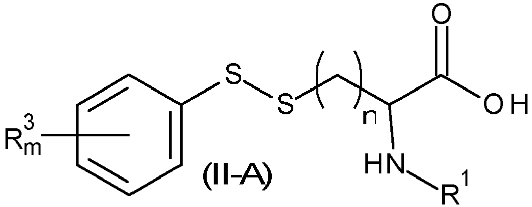 can be prepared by:
(a) reacting a compound of formula (G) 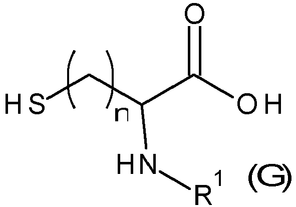 with a compound of formula (VI) 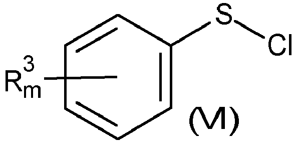 so as to obtain a compound of formula (II-A),
   wherein n, m, R¹, and R³ are as defined herein.

Amino acid derivatives of the present invention having formula (II-B) 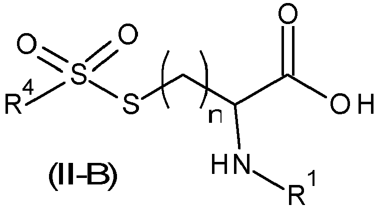 can be prepared by:
(b) reacting a compound of formula (G) 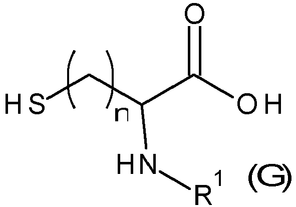 with nitrous acid so as to obtain a compound of formula (VII) 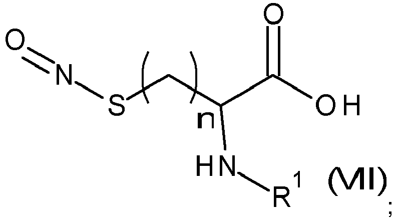 and
(c) reacting the compound of formula (VII) with a salt comprising the anion of formula (VIII) 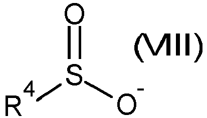
so as to obtain a compound of formula (II-B),
wherein n, R¹ and R⁴ are as defined herein.

A particular method for preparing a compound of formula (I) comprises reacting a compound of formula (II) with phosgene, diphosgene or triphosgene so as to obtain the compound of formula (I), wherein in the formulae (I) and (II) n, R¹ and R² are as described herein.

Said particular method for preparing compound of formula (I) is expediently performed under a chemically inert atmosphere such as a nitrogen atmosphere. The compound of formula (II) is expediently used in the form of a suspension in an organic solvent. It is preferred that the organic solvent is a solvent such as tetrahydrofuran (THF), wherein a compound of formula (II) is insoluble, while the NCA thereof (i.e. the corresponding compound of formula (I)) is higly soluble. The amount of solvent depends on the scale and is typically in the range of 1 g compound of formula (I) per about 1-500 ml solvent. The reaction is expediently carried out at a temperature in the range of about 50-90°C, in particular in the range of about 60-80°C and preferably at about 70°C. The amount of phosgene, diphosgene or triphosgene added is expediently in the range of 1.1-2.0 mol, in particular 1.3-1.8 mol, preferably 1.5-1.6 mol, phosgene equivalents per mol compound of formula (II) used. Reaction is typically completed within 1-3 h after addition of the phosgene, diphosgene or triphosgene.

The purification of the compound of formula (I) from the reaction mixture may include steps such as:
(i) the removal of gaseous impurities (e.g. HCl and residual phosgene) by a dry inert gas such as nitrogen gas through the reaction mixture for a sufficient amount of time (typically 2-3 h);
(ii) the removal of gaseous impurities and volatile solvent by applying a vacuum;
(iii) one or more than one steps of suspending the to be purified product in a solvent of the compound of formula (I) such as THF and precipitating the compound of formula (I) by addition of a non-solvent such as hexane; said suspension/precipitation steps are expediently carried out under an inert gas (such as nitrogen) atmosphere; typically about 1-10 parts in weight of the non-solvent is used relative to the total weight of the to be purified product and the solvent.

The present disclosure also provides an amino acid derivative of formula (I) 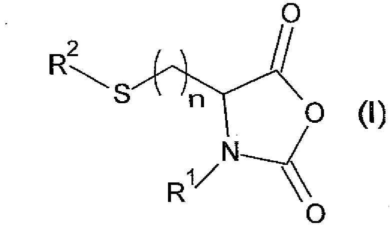 wherein
n is 1 or 2;
R¹ is H, C₁-C₄-alkyl, C₂-C₄-alkenyl or C₂-C₄-alkynyl; and
R² is a group of formula (A) 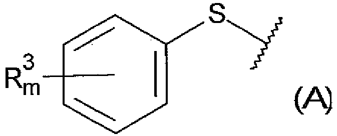 wherein
   m is 1, 2, 3, 4 or 5; and
   each R³ is independently selected from halogen, nitro, cyano and C₁-C₄-alkanoyl. In a particular subgroup of these amino acid derivatives, each R³ is independently selected from fluoro, chloro and bromo. In a further particular subgroup of these amino acid derivatives, m is 5; and R³ is chloro. In a further particular subgroup of these amino acid derivatives, m is 1, 2 or 3; and R³ is fluoro.

The present disclosure also provides an amino acid derivative of formula (II) 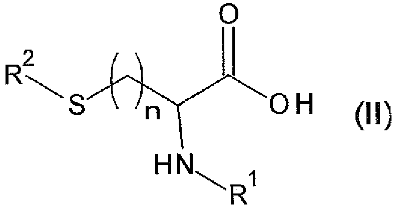 wherein
n is 1 or 2;
R¹ is H, C₁-C₄-alkyl, C₂-C₄-alkenyl or C₂-C₄-alkynyl; and
R² is a group of formula (A) 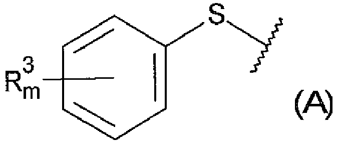 wherein
   m is 1, 2, 3, 4 or 5; and
   each R³ is independently selected from halogen, nitro, cyano and C₁-C₄-alkanoyl. In a particular subgroup of these amino acid derivatives, each R³ is independently selected from fluoro, chloro and bromo. In a further particular subgroup of these amino acid derivatives, m is 5; and R³ is chloro. In a further particular subgroup of these amino acid derivatives, m is 1, 2 or 3; and R³ is fluoro.

The groups of formula (A) described herein are preferably selected from groups of formulae (A-1), (A-2), (A-3), (A-4) and (A-5) 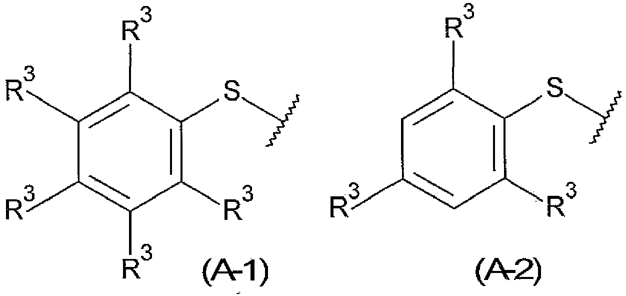 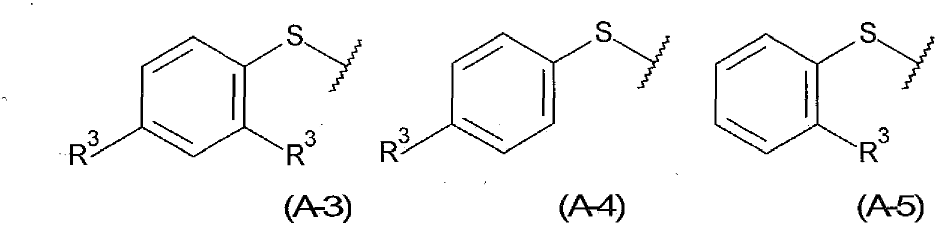 wherein each R³ is independently selected from halogen, nitro, cyano and C₁-C₄-alkanoyl, in particular from fluoro, chloro and bromo, preferably is fluoro or chloro. Particular groups of formula (A) include (2,3,4,5,6-pentafluorophenyl)sulfanyl, (2,4,6-trifluorophenyl)sulfanyl, (2,4-difluorophenyl)sulfanyl, (2-fluorophenyl)sulfanyl, (4-fluorophenyl)sulfanyl, (2,3,4,5,6-pentaclorophenyl)sulfanyl, (2,4,6-trichlorophenyl)sulfanyl, (2,4-dichlorophenyl)sulfanyl, (2-chlorophenyl)sulfanyl and (4-chlorophenyl)sulfanyl; and more particularly (2,3,4,5,6-pentafluorophenyl)sulfanyl, (2,4,6-trifluorophenyl)sulfanyl, (4-fluorophenyl)sulfanyl, (2,3,4,5,6-pentaclorophenyl)sulfanyl, (2,4,6-trichlorophenyl)sulfanyl and (4-chlorophenyl)sulfanyl.

The present disclosure also provides an amino acid derivative of formula (I) 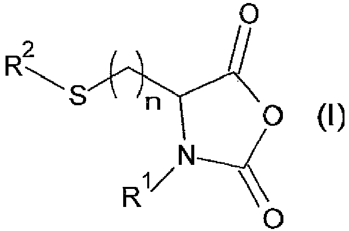 wherein
n is 1 or 2;
R¹ is H, C₁-C₄-alkyl, C₂-C₄-alkenyl or C₂-C₄-alkynyl; and
R² is a group of formula (B) 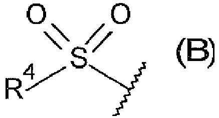 wherein
   R⁴ is selected from R^{a}-C₁-C₁₆-alkyl, R^{a}, C₁-C₁₆-alkyl, C₂-C₁₆-alkenyl and C₂-C₁₆-alkynyl, wherein the C₁-C₁₆-alkyl, C₂-C₁₆-alkenyl and C₂-C₁₆-alkynyl are unsubstituted or carry 1, 2 or 3 substituents R^{b};
   R^{b} is independently selected from halogeno, cyano, nitro, hydroxyl and thiol;
   R^{a} is selected from:
      (i) phenyl,
      (ii) 5- or 6-membered heteroaromatic monocyclic radicals having 1, 2, 3 or 4 heteroatoms as ring members which are independently selected from O, S and N,
      (iii) 8- to 10-membered heteroaromatic bicarbocyclic radicals having 1, 2, 3 or 4 heteroatoms as ring members which are independently selected from O, S and N, and
      (iv) 8- to 10-membered aromatic bicarbocyclic radicals,
      wherein said radicals (i) to (iv) are unsubstituted or carry 1, 2, 3 or 4 substituents R^{c}, and
   R^{c} is independently selected from halogeno, cyano, nitro, hydroxyl, C₁-C₄-alkyl, C₁-C₄-alkoxy, C₂-C₄-alkenyl, C₂-C₄-alkynyl and C₁-C₄-alkanoyl. In a particular subgroup of these amino acid derivatives, R⁴ is selected from R^{a} and C₁-C₁₆-alkyl, preferably R^{a}-C₁-C₈-alkyl, wherein R^{a} is as defined herein; and in particular R⁴ is unsubstituted benzyl or benzyl that carries 1, 2, 3 or 4 substituents R^{c}, wherein R^{c} is as defined herein. In a further particular subgroup of these amino acid derivatives, R⁴ is C₁-C₁₆-alkyl, preferably C₁-C₈-alkyl; and in particular R⁴ is selected from ethyl, butyl, hexyl and octyl; specifically from ethyl, n-but-1-yl and n-hex-1-yl. In a further particular subgroup of these amino acid derivatives, R⁴ is C₂-C₁₆-alkenyl, preferably C₂-C₈-alkenyl; and in particular R⁴ is selected from vinyl, butenyl, hexenyl and octenyl; specifically from vinyl, n-but-3-enyl and n-hex-5-enyl, wherein these residues may unsubstituted or partly or completely halogenated.

The present disclosure also provides an amino acid derivative of formula (II) 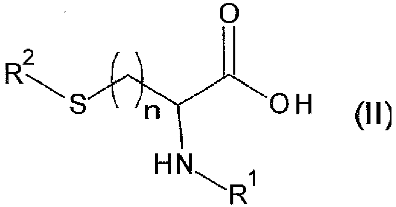 wherein
n is 1 or 2;
R¹ is H, C₁-C₄-alkyl, C₂-C₄-alkenyl or C₂-C₄-alkynyl; and
R² is a group of formula (B) 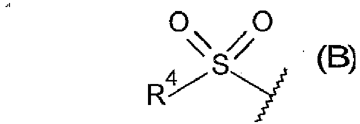 wherein
   R⁴ is selected from R^{a}-C₁-C₁₆-alkyl, R^{a}, C₁-C₁₆-alkyl, C₂-C₁₆-alkenyl and C₂-C₁₆-alkynyl, wherein the C₁-C₁₆-alkyl, C₂-C₁₆-alkenyl and C₂-C₁₆-alkynyl are unsubstituted or carry 1, 2 or 3 substituents R^{b};
   R^{b} is independently selected from halogeno, cyano, nitro, hydroxyl and thiol;
   R^{a} is selected from:
      (i) phenyl,
      (ii) 5- or 6-membered heteroaromatic monocyclic radicals having 1, 2, 3 or 4 heteroatoms as ring members which are independently selected from O, S and N,
      (iii) 8- to 10-membered heteroaromatic bicarbocyclic radicals having 1, 2, 3 or 4 heteroatoms as ring members which are independently selected from O, S and N, and
      (iv) 8- to 10-membered aromatic bicarbocyclic radicals,
      wherein said radicals (i) to (iv) are unsubstituted or carry 1, 2, 3 or 4 substituents R^{c}, and
   R^{c} is independently selected from halogeno, cyano, nitro, hydroxyl, C₁-C₄-alkyl, C₁-C₄-alkoxy, C₂-C₄-alkenyl, C₂-C₄-alkynyl and C₁-C₄-alkanoyl. In a particular subgroup of these amino acid derivatives, R⁴ is selected from R^{a} and C₁-C₁₆-alkyl, preferably R^{a}-C₁-C₈-alkyl, wherein R^{a} is as defined herein; and in particular R⁴ is unsubstituted benzyl or benzyl that carries 1, 2, 3 or 4 substituents R^{c}, wherein R^{c} is as defined herein. In a further particular subgroup of these amino acid derivatives, R⁴ is C₁-C₁₆-alkyl, preferably C₁-C₈-alkyl; and in particular R⁴ is selected from ethyl, butyl, hexyl and octyl; specifically from ethyl, n-but-1-yl and n-hex-1-yl. In a further particular subgroup of these amino acid derivatives, R⁴ is C₂-C₁₆-alkenyl, preferably C₂-C₈-alkenyl; and in particular R⁴ is selected from vinyl, butenyl, hexenyl and octenyl; specifically from vinyl, n-but-3-enyl and n-hex-5-enyl, wherein these residues may unsubstituted or partly or completely halogenated.

The protected thiol groups described herein, i.e. the disulfide group of formula (A') and the thiolsulfonate groups of formula (B'), are relatively stable under acidic conditions, under basic conditions and also against different amines, while being highly reactive against other thiols. The corresponding protecting groups of formulae (A) and (B) are therefore particularly useful in a method of intermediate thiol protection by protecting at least one thiol group of a (first) thiol compound having at least one thiol group and at least one functional group other than a thiol group ("non-thiol functional group"), wherein a chemical reaction is to be effected on at least one "non-thiol functional group" of the thiol compound under conditions to which an unprotected thiol group would be chemically reactive. To this end, at least one thiol group of said (first) thiol compound is protected with a group of formula (A) or a group of formula (B) as described herein prior to the chemical reaction on the non-thiol functional group. Then said chemical reaction is effected under conditions to which the protected thiol group is chemically inert but to which an unprotected thiol group would be chemically reactive. Subsequently, at least part of the protected thiol groups can be reacted with a second thiol compound so as to obtain a disulfide of the first thiol compound and the second thiol compound. Alternatively, at least part or all of the protected thiol groups can be deprotected so as to obtain free thiol groups (-SH). In the method of intermediate thiol protection, the protection with groups selected from groups of formulae (A) and (B) prior effecting the chemical reaction on the non-thiol group(s) as well as the subsequent reaction with the thiol group(s) of a second thiol compound or deprotection can be effected on basically all thiol groups of the first thiol compound.

According to one group of the methods of intermediate thiol protection described herein, the first thiol compound is a thiol-protected amino acid derivative of formula (IV) 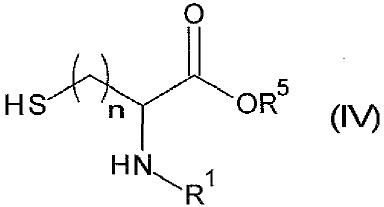 wherein
n is 1 or 2;
R¹ is H, C₁-C₄-alkyl, C₂-C₄-alkenyl or C₂-C₄-alkynyl; and
R⁵ is H, C₁-C₄-alkoxy or a carboxy-protecting group. In a particular subgroup of these embodiments, n is 1; R¹ is H, C₁-C₄-alkyl, C₂-C₄-alkenyl or C₂-C₄-alkynyl, and preferably is H, methyl, ethyl, propargyl or but-3-enyl; and R⁶ is a group of formula (A) or formula (B) as described herein. In a more particular subgroup of these methods, R⁶ is a group of formula (A) as described herein. In an alternative particular subgroup of these methods, R⁶ is a group of formula (B) as described herein. In a preferred subgroup of these methods, R¹ is H, methyl, ethyl, propargyl or but-3-enyl.

According to an alternative group of the methods of intermediate thiol protection described herein, the first thiol compound is a peptide (e.g. a polypeptide) comprising at least one amino acid residue of formula (C) 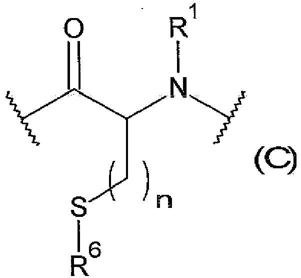 wherein
n is 1 or 2;
R¹ is H, C₁-C₄-alkyl, C₂-C₄-alkenyl or C₂-C₄-alkynyl; and
R⁶ is independently selected from H, a group of formula (A) and a group of formula (B) as described herein. In a particular subgroup of these methods, n is 1; R¹ is H, C₁-C₄-alkyl, C₂-C₄-alkenyl or C₂-C₄-alkynyl; and R⁶ is a group of formula (A) or formula (B) as described herein. In a more particular subgroup of these methods, R⁶ is a group of formula (A) as described herein. In an alternative particular subgroup of these methods, R⁶ is a group of formula (B) as described herein. In a preferred subgroup of these methods, R¹ is H, methyl, ethyl, propargyl or but-3-enyl.

In one group of the methods of intermediate thiol protection described herein, at least part of the thiol groups of the first thiol compound are protected by groups formula (B) as described herein.

In an alternative group of the methods of intermediate thiol protection described herein, at least part of the thiol groups of the first thiol compound are protected by groups formula (A) as described herein. In those methods, the protection of the thiol groups may be effected by reacting the thiol compound (specifically, at least part of the free thiol groups thereof) with a compound of formula (III)

A-R⁷ (III)

wherein R⁷ is chloro and A is a group of formula (A) as defined herein.

A compound of formula (III) can be prepared using method known in the art starting from a compound of formula (III')

A-H (III')

wherein A is a group of formula (A) as defined herein (i.e. the free thiol of the group of formula (A)). For example, a compound of formula (III') can be reacted with a chlorinating agent such as sulfuryl chloride so as to obtain the thiohypochlorite of formula (III). Said reaction is typically performed in a polar organic solvent such as dichloromethane or chloroform and typically completed after about 3-12 h at room temperature.

The present disclosure also provides a method for preparing a compound of formula (II) 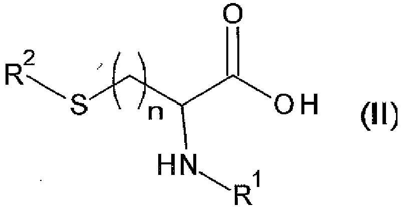
wherein n and R¹ are as defined herein, and
R² is a group of formula (A) as defined herein,
the method comprising reacting an amino acid derivative of formula (II') 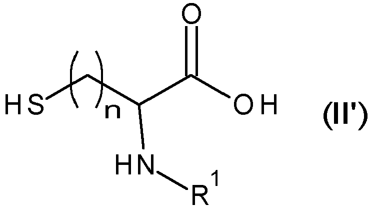 wherein n and R¹ are the same as in in the compound of formula (II),
with a compound of formula (III)

A-R⁷ (III)

wherein R⁷ is chloro and A is the same group of formula (A) as in the compound of formula (II).

The thiol groups protected with groups selected from groups of formulae (A) and (B) can be directly converted into more stable disulfides by nucleophilic substitution with soft nucleophiles. Thus, the modification of these protected thiol groups does not require an intermediate step of deprotection and the intermediate formation of free thiol groups.

The compounds of formula (I) or formula (II) described herein are useful for coupling a group of formula (II-C) 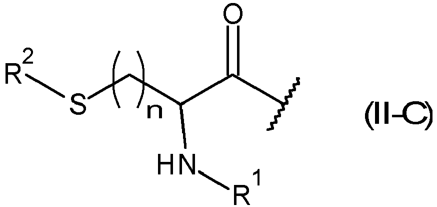 to a compound comprising a carboxyl-reactive group (e.g. an amino group);
or for coupling a group of formula (II-N) 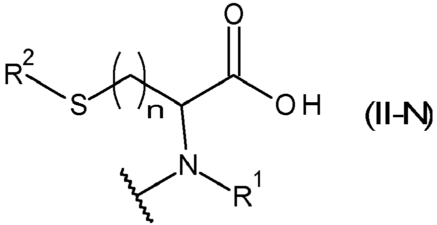 to a compound comprising an amino-reactive group (e.g. a carboxyl group);
wherein the n, R¹ and R² in formulae (I), (II), (II-C) and (II-N) are as defined herein.

The compound comprising a carboxyl-reactive group and the compound comprising an amino-reactive group are preferable selected from amino acids (including natural and non-natural amino acids) and from compounds comprising such amino acid residue such as peptides (e.g., dipeptides, oligopeptides, polypeptides).

The coupling reaction is effected by contacting a compound of formula (I) or formula (II) with the compound comprising the amino-reactive or carboxyl-reactive group, respectively, under coupling conditions.

According to one aspect, the invention provides a method for preparing a peptide (in particular a polypeptide) comprising at least one amino acid residue that carries an, optionally protected, thiol-group, wherein the method comprises reacting an amino acid derivative of formula (I) as described herein with an amino acid or with an amino acid residue of a precursor peptide under coupling conditions, thus obtaining a peptide comprising at least one thiol-protected amino acid residue.

According to a further aspect, the invention provides a method for preparing a peptide (in particular a polypeptide) comprising at least one amino acid residue that carries an, optionally protected, thiol-group, wherein the method comprises reacting an amino acid derivative of formula (II) as described herein with an amino acid or with an amino acid residue of a precursor peptide under coupling conditions, thus obtaining a peptide comprising at least one thiol-protected amino acid residue.

Thus, the peptide will be formed by a covalent coupling of the thiol-protected amino acid derivative with the amino acid so as to form a dipeptide, or by a covalent coupling of the thiol-protected amino acid derivative to the amino acid residue of the precursor peptide. Preferably, the thiol-protected amino acid derivative is coupled to a terminal amino acid residue (in particular the N-terminal amino acid residue) of the precursor peptide so as to result in an elongation of the peptide chain.

According to one group of embodiments of such method for preparing a peptide, additionally, at least part of the thiol-protected amino acid residues of the resulting peptide are reacted with a thiol compound, thus obtaining disulfide adducts of said amino acid residues and the thiol compound. The thiol compound may comprise one, two or more than two thiol groups. Where the thiol compound comprises two or more thiol group, it can be reacted with the (protected) thiol groups of the resulting peptides so as to cross-link said peptides.

According to a further group of embodiments of such method for preparing a peptide, additionally, at least part of the thiol-protected amino acid residues of the resulting peptide are deprotected.

In the method of the invention for preparing a peptide (in particular a polypeptide) described herein, amino acid or peptide to be reacted with the amino acid derivative of formula (I) or formula (II) can be is stably associated with the surface of a polymeric support material.

Methods for preparing polymers by coupling amino groups with amino-reactive groups and carboxyl groups with carboxyl-reactive groups, and specifically method for preparing peptides (in particular polypeptides) by coupling amino groups with carboxyl groups, are known in the art and applicable in the context of the present invention. Compounds of formula (I) as described herein are particularly useful for preparing polypeptides via ring-opening polymerization (ROP) using methods known in the art, for example methods as described by Hadjichristidis et al. (Chem. Re. 2009, 209: 5528-5578) and the literature described therein.

Thus, a compound of formula (I) can be used in a method of preparing a polymer (such as a polypeptide) via ROP, wherein the method comprises reacting the compound of formula (I) with either a further compound of formula (I) or with another amino acid NCA under coupling conditions. Typically, such method of preparing a polymer via ROP is carried out in a polar organic solvent such as dimethylformamide (DMF). The reaction is typically carried out at a temperature between 5-40°C, in particular between 10-30°C or more specifically at about room temperature. The ratio of NCA (e.g. compound of formula (I)) to solvent is typically in the range of 50-500 mg of the NCA per ml solvent. For the preparation of high molecular weight polymers (i.e. polymers having a number average molecular weight of 50,000 g/mol or more), the ratio of NCA to solvent is preferably, in the range of 50-100 mg NCA per ml solvent. For the preparation of low molecular weight polymers (i.e. polymers having a number average molecular weight of less than 50,000 g/mol), the ratio of NCA to solvent is preferably, in the range of 100-500 mg NCA per ml solvent. The ROP is initiated by addition of an initiator that is expediently added after the NCA is completely dissolved in the solvent. Useful initiators are known in the art (cf., e.g., Hadjichristidis et al., Chem. Re. 2009, 209: 5528-5578) and include primary amines and specifically amines comprising alkenyl, alkynyl, azide or biotin groups. A preferred initiator is neopentylamine because it allows for precise determination of chain length by endgroup analysis. By applying a constant pressure of an inert gas such as nitrogen impurities can be prevented from entering the reaction during polymerization, while carbon dioxide liberated during polymerization can be allowed to escape. To purify the polymer (e.g. polypeptide) after ROP, the product is precipitated with a non-solvent such as diethylether or cyclohexane. Residual polar organic solvent (e.g. DMF) can be removed by repeated precipitation with the non-solvent. Finally, residual non-solvent can be removed from the precipitate by drying (typically in high vacuum such as at about 0.1 Pa), dialysis or ultrafiltration and/or by lyophilization (in particular if the polymer is soluble in water or 1,4-dioxane).

The thiol-protecting groups of formulae (A) and (B), and in particular the thus thiol-protected amino acid derivatives of formulae (I) and (II) described herein are useful for preparing polymers, including homopolymers and copolymers and in particular block copolymers, comprising amino acid monomeric units having a thiol group, such as a unit of formula (C) 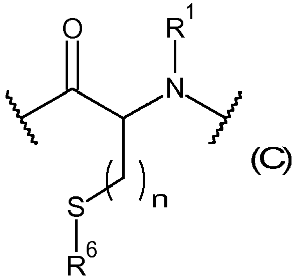 wherein n and R¹ are as defined herein; and R⁶ is independently selected from H, a group of formula (A) and a group of formula (B) as described herein.

The term "block copolymer" as used herein refers to a copolymer comprising two or more different monomers in a linear block structure. For example, a block copolymer can be built of two (diblock copolymer), three (triblock copolymer) or more than three (multiblock copolymer) different polymeric blocks which are covalently linked so as to form a linear block structure. Particular examples of block copolymers are block copolypeptides which are exclusively build from peptide blocks, as termed "block-copolypeptides".

The polymeric blocks of a block copolymer may have different properties, e.g., with regard to hydrophilicity/hydrophobicity, overall charge (anionic/cationic) and functionality/reactivity (e.g. thiol-reactive blocks, blocks with carboxy functionality and blocks with amino functionality).

The term "carboxy functionality" as used herein refers to the capability of reacting with compounds carrying amino or hydroxyl groups so as to form esters or amides, respectively. The term "amino functionality" as used herein refers to the capability of reacting with compounds carrying carboxy groups so as to form amides, respectively.

The term "hydrophobic" as used herein refers to molecules or polymeric blocks that are basically insoluble in water. Specifically, less than 100 mg/ml, more preferably less than 50 mg/ml and even more preferably less than 10 mg/ml of a hydrophobic molecule (or a molecule corresponding to a hydrophobic polymeric block) will dissolve in water at 25°C and pH 7.0. Conversely, the term "hydrophilic" as used herein refers to molecules or polymeric blocks that are water soluble, or generally capable of adsorbing water.

According to one aspect, the invention provides a block copolymer comprising at least one hydrophilic block and at least one thiol-reactive block as described herein.

The block copolymers of the invention, comprising a hydrophilic block and a thiol-reactive block as described herein, can comprise at least one further polymeric block. Said further polymeric block(s) may be selected from polycationic polymeric blocks and hydrophobic polymeric blocks.

According to particular groups of embodiments, the block copolymer of the invention comprises a linear sequence of polymeric blocks selected from:
(i) a hydrophilic block followed by a thiol-reactive block
(ii) a hydrophilic block followed by a thiol-reactive block followed by a hydrophobic block,
(iii) a hydrophilic block followed by a thiol-reactive block followed by a polycationic block,
(iv) a hydrophilic block followed by a hydrophobic block followed by a thiol-reactive block, and
(v) a hydrophilic block followed by a polycationic block followed by a thiol-reactive block,
wherein
said hydrophilic block can consist of 10-1000 monomeric units and preferably consists of 50-400 monomeric units,
said thiol-reactive block can consist of 1-1000 monomeric units and preferably consists of 2-400, 5-100 or, in particular, 10-50 monomeric units,
said polycationic block can consist of 1-1000 monomeric units and preferably consists of 2-400, 5-100 or, in particular, 10-50 monomeric units, and
said hydrophobic block can consist of 1-1000 monomeric units and preferably consists of 2-400, 5-100 or, in particular, 10-50 monomeric units.

The term "hydrophilic polymeric blocks" (in short: "hydrophilic blocks") as used herein refers to linear hydrophilic polymeric blocks of 10-1000, preferably 50-400, monomeric units. The hydrophilic blocks of the block copolymer of the invention may be selected, for example, from hydrophilic, polypeptide moieties, poly(ethylene) glycol moieties, hydrophilic polysaccharide moieties, optionally substituted polymethacrylic acid moieties (e.g. poly(N-(2-hydroxypropyl)methacrylamide), pHPMA), optionally substituted polyacrylic acid moieties (e.g. polyhydroxyethylmethacrylate, pHEMA), polyvinylalcohol moieties, polyvinylpyrrolidone moieties and poly(oxazoline) moieties (POx). The hydrophilic blocks of the block copolymer of the invention can be *inter alia* polycationic and polyanionic blocks as described herein.

For block copolymers for pharmaceutical use, hydrophilic blocks such as poly(ethylene) glycol moieties, poly(oxazoline) moieties and hydrophilic polypeptide moieties are particularly preferred.

Hydrophilic polypeptide moieties may be built from monomeric units selected from proteinogenic and non-proteinogenic amino acid residues. Hydrophilic polypeptide moieties are preferably selected from polymers of natural amino acids, in particular those occurring naturally in the body of the subject to be administered the block copolymer comprising said hydrophilic polypeptide moiety. Specific examples include polylysine (PLys) and poly(glutamic acid) (PGlu), and in particular polysarcosine (PSar).

Thiol-reactive polymeric blocks (in short: "thiol-reactive blocks") are polymeric blocks comprising thiol groups which may react with thiols so as to form disulfides. The thiol groups of the thiol-reactive blocks may be at least partially protected with thiol-protecting groups such as groups of formulae (A) or (B) described herein. Specifically, thiol-reactive blocks according to the present invention are linear polymers of 1-1000, preferably 2-400, 5-100, and in particular 10-50, monomeric units of formula (C) 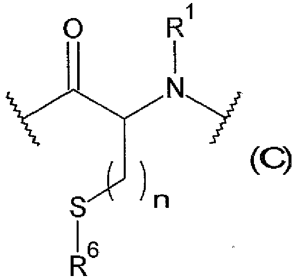 wherein n and R¹ are as defined herein; and R⁶ is independently selected from H, a group of formula (A) and a group of formula (B) as described herein, wherein in at least one monomeric unit of formula (C) R⁶ is a group of formula (A) or formula (B).

According to a particular group of embodiments, the block copolymer of the invention is a compound of formula (V) 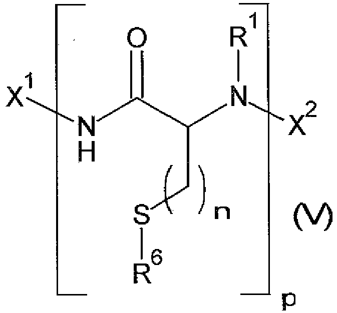
wherein X¹ is said hydrophilic block;
p is 1-1000, preferably 2-400, 5-300, and in particular 10-100;
n and R¹ are as defined herein;
R⁶ is independently selected from H, a group of formula (A) and a group of formula (B) as described herein, wherein in at least one monomeric unit of formula (V) R⁶ is a group of formula (A) or formula (B); and
X² is H or at least one further block of the block copolymer.

The hydrophobicity of a thiol-reactive block of a block copolymer according to the invention depends on the number of monomeric units, wherein R⁶ is selected from a thiol-protecting group of formula (A) and a thiol-protecting group of formula (B), relative to the total number of monomeric units, and on the hydrophobicity of said thiol-protecting groups. The terms "hydrophobic (polymeric) block" and "hydrophilic (polymeric) block" as used herein therefore do not refer to the thiol-reactive blocks described herein.

The term "polycationic polymeric block" (in short: "polycationic block") as used herein refers to linear polycationic polymeric blocks of 1-1000, preferably 2-400, 5-100, and in particular 10-50, monomeric units.

Examples of polycationic blocks include polycationic polypeptide moieties build from monomeric units selected from proteinogenic and non-proteinogenic cationic amino acid residues. Polycationic polypeptide moieties are preferably selected from polymers of natural amino acids, in particular those occurring naturally in the body of the subject to be administered the block copolymer comprising said hydrophilic polypeptide moiety. Specific examples include polyarginine (pArg), polyhistidine (pHis) and polylysine (pLys). Thus, according to particular embodiments, the polycationic block comprised by a block copolymer of the invention may be a block having monomeric units of formula (D) 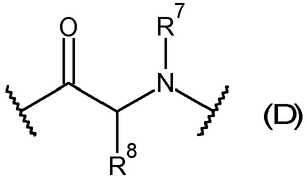 wherein R⁷ is H or C₁-C₄-alkyl; and R⁸ is independently selected from 4-aminobutyl, 3-guanidinopropyl and 1H-imidazol-4-ylmethyl.

The term "polyanionic polymeric block" (in short: "polyanionic block") as used herein refers to linear polyanionic polymeric blocks of 1-1000, preferably 2-400, 5-100, and in particular 10-50, monomeric units.

Examples of polyanionic blocks include polyanionic polypeptide moieties build from monomeric units selected from proteinogenic and non-proteinogenic anionic amino acid residues. Polyanionic polypeptide moieties are preferably selected from polymers of natural amino acids, in particular those occurring naturally in the body of the subject to be administered the block copolymer comprising said hydrophilic polypeptide moiety. Specific examples include polyaspartic acid (pAsp) and polyglutamic acid (pGlu).

The term "hydrophobic polymeric blocks" (in short: "hydrophobic blocks") refers to linear hydrophobic polymeric blocks of 1-1000, preferably 2-400, 5-100, and in particular 10-50 monomeric units.

Examples of hydrophobic blocks include hydrophobic polypeptide moieties build from monomeric units selected from proteinogenic and non-proteinogenic cationic amino acid residues. Hydrophobic polypeptide moieties are preferably selected from polymers of natural amino acids, in particular those occurring naturally in the body of the subject to be administered the block copolymer comprising said hydrophilic polypeptide moiety. Specific examples include polyalanine (PAla), polyphenylalanine (PPhe), polyleucine (PLeu), polynorleucine (PNLeu), and hydrophobic derivatives thereof.

Expediently, the polymeric blocks of the block copolymer of the invention are linked to each other by carboxamide bonds (e.g. peptide bonds). Where the polymeric blocks are polypeptide moieties, said polymeric blocks can be synthesized by ring opening polymerization of NCAs or successive coupling of amino acid residues in N->C or C->N direction on a solid phase.

Thus, in a further aspect, the invention provides a method for preparing a block copolymer, wherein the method comprises:
(i) providing a hydrophilic polymer of 10-1000 monomeric units that carries at least one terminal primary or secondary amino group; and
(ii) reacting a thiol-protected amino acid derivative of formula (I) or of formula (II) as defined herein with the terminal amino group of the hydrophilic polymer,
thus forming a polymer that carries a terminal primary or secondary amino group. Preferably, in step (ii) of the method, a compound of formula (I) is used. Where the primary or secondary amino group is to be reacted with a compound of formula (II), the carboxyl group of the compound of formula (I) is expediently activated beforehand. The carboxyl group of the compound of formula (II) can be activated by applying methods known in the art such as activating the carboxyl group by converting it into an acyl chloride, a 4-nitrophenyl ester or an N-hydroxysuccinimide ester.

The method for preparing a block copolymer according to the invention can further comprise:
(iii) reacting a thiol-protected amino acid derivative of formula (I) or of formula (II) as described herein with the terminal amino group of the polymer obtained in step (ii); and
(iv) optionally repeating the reacting of a thiol-protected amino acid derivative of formula (I) with the obtained polymer once or more than once.

In a further aspect, the invention provides a core-shell particle comprising:
a multitude of block copolymers (comprising at least one hydrophilic block and at least one thiol-reactive block) as described herein, and
at least one cargo molecule, such as a pharmaceutically active agent and imaging agent (e.g. a fluorescent dye, enzyme of radio-labelled substance) or a polynucleotide, encapsulated therein;
wherein the hydrophilic blocks of the block copolymers are located in the shell region of the particle, and the thiol-reactive blocks of the block copolymers as well as the cargo molecule(s) are located in the core region of the particle.

According to one group of embodiments, the core-shell particle of the invention comprises block copolymers which comprise a hydrophilic block followed by a thiol-reactive block, optionally followed by a hydrophobic block.

Particles having a hydrophobic core and a hydrophilic shell (herein also referred to as "PeptoMicelles") can be formed by self-aggregation of amphiphilic block copolymers of the invention. "Amphiphilic block copolymers" are block copolymers comprising a hydrophilic region (such as the region including the hydrophilic block) and a hydrophobic region (such as the region including the thiol-reactive block and, if present, the hydrophobic block). Amphiphilic block copolymers can self-aggregate in polar media so as to form particles having a hydrophobic core and a hydrophilic shell. Suitable polar media for preparing core-shell particles by inducing self-aggregation of block copolymers of the invention include, but are not limited to, water, aqueous media, dimethyl sulfoxide, dimethylformamide, alcohols (such as methanol, ethanol and 1-propanol) and mixtures thereof.

Thus, a core-shell particle of the invention can be formed by a method comprising:
(i) preparing a fine dispersion of amphiphilic block copolymers (comprising at least one hydrophilic block and at least one thiol-reactive block) as described herein in a polar medium so as to allow self-aggregation of the block copolymer into particles having a hydrophobic core and a hydrophilic shell;
(ii) encapsulating cargo molecules in the particles or conjugating cargo molecules with the particles.

In this another methods described herein, fine dispersions of amphiphilic block copolymers in a liquid medium can be prepared by methods known in the art such as film rehydration and solvent exchange.

Hydrophobic cargo molecules, such as hydrophobic pharmaceutically active agents, can be encapsulated by hydrophobic interactions with the core region of the particles. To this end, the hydrophobic cargo molecules can be added to polar medium, wherein the self-aggregation of the amphiphilic copolymers forming the particles takes place, or can be mixed with a dispersion of particles in a polar medium.

At least part of, or all, the cargo molecules can be covalently coupled to the thiol-reactive blocks of the block copolymers of the core-shell particles of the invention. Specifically, a cargo molecules can be covalently linked to the thiol-reactive block such that at least one disulfide bond is formed between a (protected) thiol group of the thiol-reactive block and, if present, a thiol group of the cargo molecule. Alternatively, a cargo molecules can be covalently linked to the thiol-reactive block such that at least one disulfide bond is formed between a (protected) thiol group of the thiol-reactive block and a thiol group of a linker molecule that carries one, two or more than two thiol groups and is attached to the cargo molecule.

Thus, in core-shell particle of the invention, the covalently bound cargo molecule and a monomer of the thiol-reactive block of a block copolymer can form the structure (E) 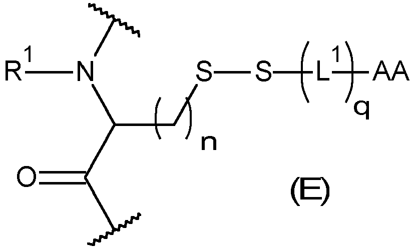 wherein
n and R¹ are as described herein;
q is 0 or 1;
L¹ is a linker; and
AA is the covalently bound cargo molecule (such as a pharmaceutically active agent).

Stabilized core-shell particles can be prepared by cross-linking the thiol-reactive blocks of the block copolymers after they have self-aggregated so as to form the core-shell structure.

The present invention provides a method for stabilizing a core-shell particle of the invention as described herein, wherein the method comprises contacting the core-shell particle with a cross-linker comprising two or more than two thiol groups.

Accordingly, in a core-shell particle of the invention, at least two monomers of the thiol-reactive blocks of the block copolymers comprised by the core-shell particle can be cross-linked via a cross-linker comprising two or more than two thiol groups.

The cross-linker used to modify the core-shell particles according to the present invention is a molecule comprising two or more than two thiol groups. Cross-linking is achieved by the formation of disulfide bonds between thiol groups of the thiol-reactive blocks of the block copolymer and thiol groups of the cross-linker. Suitable cross-linkers include, for example, hydrocarbons, carbohydrates, peptides (such as dipeptides or oligopeptides), fatty acids and derivatives thereof, which have been modified so as to comprise two or more than two thiol groups. Examples of suitable cross-linkers include peptides (such as dipeptides or oligopeptides) comprising two or more than two amino acid residues having thiol groups such as cysteine residues and homocysteine residues. Specific examples of cross-linkers include peptides (such as dipeptides or oligopeptides), wherein the terminal amino acid residues are selected from cysteine and homocysteine residues.

For example, the at least two monomers of the thiol-reactive blocks can be cross-linked via the cross-linker so as form the structure (F) 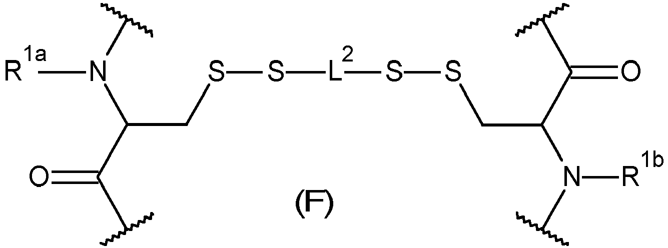 wherein R^{1a} and R^{1b} are independently selected from groups R¹ as defined herein; and L² is the cross-linker residue. The structure F is formed by cross-linking the thiol-reactive blocks with a cross-linker of formula HS-L²-SH. For example, L² can be selected from residues of hydrocarbons (e.g. C₂-C₁₆-alkylene such as -(CH₂)₆-), carbohydrates, amino acids, peptides (such as dipeptides or oligopeptides), linear polyamines and polyamine derivatives (such as -CH₂-CH(NH₂)-C(O)-(NH-CH₂-CH₂)ₙ-NH-C(O)-CH(NH₂)-CH₂-), fatty acids, and derivatives of the aforementioned.

The stabilization of the core-shell structure of the particles by cross-linking can be performed before or after the cargo molecules are encapsulated by or conjugated with the particles.

According to one particular group of embodiments (herein also referred to as "NanoPeptoGels"), the thiol-reactive blocks in the core of the core-shell particles of the invention are cross-linked so as to form a hydrophilic structure, to which hydrophilic cargo molecules are bound by physical (such as hydrophilic) interactions or chemical bonds. Such core-shell particles are formed by a method comprising:
(i) preparing a fine dispersion of amphiphilic block copolymers (comprising at least one hydrophilic block and at least one thiol-reactive block) as described herein in a polar medium so as to allow self-aggregation of the block copolymers into particles having a hydrophobic core and a hydrophilic shell;
(ii) cross-linking the thiol-reactive blocks of the block copolymers via a hydrophilic cross-linker comprising two or more than two thiol groups;
(iii) contacting the cross-linked particles with hydrophilic cargo molecules so as to form core-shell particles each comprising a multitude of the block copolymers and at least one negatively charged cargo molecule, and
(iv) optionally coupling the cargo molecules covalently to the cross-linked block copolymers,
wherein the hydrophilic blocks of the block copolymers are located in the shell region of the particle, and the cross-linked thiol-reactive blocks of the block copolymers as well as the cargo molecules are located in the core region of the particle. Cross-linking the thiol-reactive blocks with a hydrophilic cross-linker as in step (ii) stabilizes the core-shell structure of the particles formed in step (i) and converts the hydrophobic core of said particles into a hydrophilic core that binds hydrophilic cargo molecules by hydrophilic interactions.

According to one particular subgroup of NanoPeptoGels, the thiol-reactive blocks in the core of the core-shell particles of the invention are cross-linked so as to form a hydrophilic polycationic structure, to which negatively charged cargo molecules are bound by physical (such as electrostatic) interactions or chemical bonds. Such core-shell particles are formed by a method comprising:
(i) preparing a fine dispersion of amphiphilic block copolymers (comprising at least one hydrophilic block and at least one thiol-reactive block) as described herein in a polar medium so as to allow self-aggregation of the block copolymers into particles having a hydrophobic core and a hydrophilic shell;
(ii) cross-linking the thiol-reactive blocks of the block copolymers via a cationic cross-linker comprising two or more than two thiol groups;
(iii) contacting the cross-linked particles with negatively charged (anionic) cargo molecules so as to form core-shell particles each comprising a multitude of the block copolymers and at least one negatively charged cargo molecule, wherein the hydrophilic blocks of the block copolymers are located in the shell region of the particle, and the cross-linked thiol-reactive blocks of the block copolymers as well as the cargo molecules are located in the core region of the particle, and
(iv) optionally coupling the cargo molecules covalently to the cross-linked block copolymers.

Cross-linking the thiol-reactive blocks with a cationic cross-linker as in step (ii) stabilizes the core-shell structure of the particles formed in step (i) and converts the hydrophobic core of said particles into a polycationic core that complexes negatively charged cargo molecules (such as oligomers or polymers of ribonucleic or desoxyribonucleic acid, e.g., small interfering RNA, siRNA) by electrostatic interactions.

The cationic cross-linker comprising two or more than two thiol groups can be selected from oligoamines, e.g. oligoethyleneimines, spermine, which are modified so as to carry two or more thiol groups, e.g. by reacting the terminal amino groups of the oligoamine with cysteine. The thiol-reactive blocks are cross-linked by the formation of disulfide bonds between the thiol groups of the cross-linker and thiol-groups of the thiol-reactive blocks.

According to an alternative particular subgroup of NanoPeptoGels, the thiol-reactive blocks in the core of the core-shell particles of the invention are cross-linked so as to form a polyanionic structure, to which positively charged cargo molecules are bound by physical (such as electrostatic) interactions or chemical bonds. Such core-shell particles can be prepared by a method comprising:
(i) preparing a fine dispersion of block copolymers (comprising at least one hydrophilic block and at least one thiol-reactive block) as described herein in a polar medium so as to allow self-aggregation of the block copolymers into particles having a hydrophobic core and a hydrophilic shell;
(ii) cross-linking the thiol-reactive blocks of the block copolymers via an anionic cross-linker comprising two or more than two thiol groups;
(iii) contacting the cross-linked particles with positively charged (cationic) cargo molecules so as to form core-shell particles each comprising a multitude of the block copolymers and at least one positively charged cargo molecule, wherein the hydrophilic blocks of the block copolymers are located in the shell region of the particle, and the cross-linked thiol-reactive blocks of the block copolymers as well as the cargo molecules are located in the core region of the particle, and
(iv) optionally coupling the cargo molecules covalently to the cross-linked block copolymers.

Cross-linking the thiol-reactive blocks with an anionic cross-linker as in step (ii) stabilizes the core-shell structure of the particles formed in step (i) and converts the hydrophobic core of said particles into a polyanionic core that complexes positively charged cargo molecules (such as cationic peptides) by electrostatic interactions.

The anionic cross-linker comprising two or more than two thiol groups can be selected from oligo acids, e.g. oligocarboxylic acids, oligosulfonic acids, which are modified so as to carry two or more thiol groups, e.g. by reacting the terminal carboxylic groups of the oligo acid with cysteine. The thiol-reactive blocks are cross-linked by the formation of disulfide bonds between the thiol groups of the cross-linker and thiol-groups of the thiol-reactive blocks.

According to a further particular group of embodiments (herein also referred to as "PeptoPlexes"), the core-shell particle of the invention comprises block copolymers, which comprise a hydrophilic block (expediently a hydrophilic block other than a polycationic block) followed by a thiol-reactive block followed by a polycationic block, and at least one polyanionic (preferably polymeric) cargo molecule, such as a polynucleotide (RNA) or polyribonucleotide (DNA). Such core-shell particles can be prepared by a method comprising:
(i) contacting the block copolymers with the polyanionic cargo molecules so as to allow the formation of particles (PeptoPlexes) comprising at least one polyanionic cargo molecule complexed by the polycationic blocks of a multitude of block copolymers; and
(ii) cross-linking the thiol-reactive blocks of the block copolymers in the particles via a cross-linker comprising two or more than two thiol groups.

A thus formed core-shell particle comprises a multitude of the block copolymers and at least one polyanionic cargo molecule, wherein the polyanionic cargo molecule(s) as well as the polycationic blocks and the (cross-linked) thiol-reactive groups of the block copolymers are located in the core region of the particle, and the hydrophilic blocks of the block copolymers are located in the shell region of the particle. Cross-linking the thiol-reactive blocks with an anionic cross-linker as in step (ii) stabilizes the core-shell structure of the particles formed in step (i) by the complexation of polyanionic cargo molecules by the polycationic blocks of the block copolymers via electrostatic interactions. Step (i) is expediently performed preparing a fine dispersion of the block copolymers and the polyanionic cargo molecules in a polar medium. For example, said polar medium can be selected from water, aqueous media, dimethyl sulfoxide, dimethylformamide, alcohols (such as methanol, ethanol and 1-propanol) and mixtures thereof.

The thiol-reactive blocks of the block copolymers of the invention can be reacted with compounds comprising one thiol group such as, for example, 2-aminoethanediol, so as to reduce the number of activated thiol groups (i.e. thiol groups protected with a group of formula (A) or formula (B) as described herein) within the block copolymers by forming more disulfide bonds which are less reactive against free thiol groups. Thus reducing the number of activated thiol groups within a core-shell particle of the invention will reduce the degree of possible cross-linking of the block copolymers comprised by the core-shell particle.

The core-shell particles of the invention provide several advantages. They can be covalently modified under relatively mild conditions, for example to stabilize the particles via cross-linking, add functional groups, covalently couple the cargo, etc. The core-shell particles (in particular when stabilized via cross-linking) are stable in aqueous solutions such as pharmaceutically acceptable carrier solutions and bodily fluids. And they release their cargo and can be degraded after having been taken up by a cell in a specific intracellular compartment. Moreover, the core-shell particles of the invention can be produced from biodegradable block-copolymers which are partially or entirely based on monomeric units as found in the human and/or animal body and thus, when degraded in the subject's body, do not form extraneous compounds which might be immunogenic and/or cause other undesirable side effects.

The present invention further provides a composition comprising a core-shell particle of the invention as described herein and a pharmaceutically acceptable carrier. Pharmaceutically acceptable carriers are known in the art. Expediently, the carrier is chosen to be suitable for the intended way of administration which can be, for example, intravascular, subcutaneous or, most commonly, intravenous injection.

For example, the pharmaceutically acceptable carriers can be selected from pharmaceutically acceptable solvents or solvent mixtures such as water or aqueous solutions (e.g. saline).

### EXAMPLES

### 1) Preparation of thiol-protected amino acid derivatives of formula (II)

### a) ((Pentafluorophenyl)disulfanyl)cysteine [2-amino-3-(2,3,4,5,6-pentafluorophenyl)disulfanyl-propanoic acid]

### Pentafluorophenylthiohypochlorite:

4.56 ml sulfuryl chloride were added to a solution of 5 ml pentafluorothiophenol in 75 ml dichloromethane while stirring at 0°C. The resulting solution was stirred over night. The dichloromethane was distilled off at 45°C. Then, residual solvent and sulfuryl chloride were removed *in vacuo* which yielded 8.35 g (∼98%) product in the form of an orange liquid.

### ((Pentafluorophenyl)disulfanyl)cysteine:

Cysteine hydrochloride was recrystallized from methanol by adding diethylether to increase purity of the cysteine. 3.02 g pentafluorophenylthiohypochlorite were added to a suspension of 1.35 g cysteine hydrochloride in 35 ml acetic acid and stirred for 5 min. Further 23 ml acetic acid was added when solidification occurred so that the solid was dissolved. The resulting yellow solution was heated for 3.5 h at 70°C resulting in a progressive decolorization of the solution. The solvent was distilled off by means of a rotary evaporator *in vacuo.* The colorless to slightly yellowish distillation residue was suspended in 58 ml chloroform and filtered. The solid filtration residue was suspended in 46 ml chloroform and filtered. The resulting filtration residue was dried *in vacuo* which yielded 2.7 g (∼98%) colorless solid product.

### b) ((2-Fluorophenyl)disulfanyl)cysteine [2-amino-3-(2-fluorophenyl)disulfanyl-propanoic acid]

### 2-Fluorophenylthiohypochlorite:

5 ml 2-Fluorothiophenol were dissolved in 95 ml dichloromethane and cooled to 0°C. 5.65 ml sulfurylchloride were added dropwise and the solution was stirred over night. The solvent and excess sulfurylchloride were removed *in vacuo* which yielded 7.94 g product.
¹H NMR (400MHz, CDCl₃) ∂[ppm] = 7.71 (dt, J=1.7, 7.5, 1H), 7.51-7.41 (m, 2H), 7.25-7.13 (m, 2H).
¹⁹F NMR (400MHz, CDCl₃) ∂[ppm] = -105.76-(-105.88) (m, 1F).

### ((2-Fluorophenyl)disulfanyl)cysteine:

7.94 ml 2-Fluorophenylthiohypochlorite were mixed with 5 ml trifluoroacetic acid (TFA) and stirred vigorously. 3.68 g Cysteine hydrochloride were dissolved in 40 ml TFA. The solution of cysteine hydrochloride was slowly added to the solution of 2-fluorophenylthiohypochlorite at a rate of -28 ml/min. The TFA was removed *in vaccuo.* The product was purified by: dissolving it in 1 ml TFA and 10 ml methanol, adding 100 ml chloroform, removing any precipitate by filtration, concentrating the filtrate *in vaccuo,* precipitating the product by adding diethylether, dissolving the precipitated product in 10 ml methanol, adding 100 ml chloroform, removing any precipitate by filtration, concentrating the filtrate *in vaccuo* and precipitating the product by adding diethylether. 0.99 g ((2-fluorophenyl)disulfanyl)cysteine was obtained in the form of its TFA salt.
¹H NMR (400MHz, MeOD) ∂[ppm] = 7.75 (dt, J=1.7, 7.6, 1H, arom.), 7.49-7.41 (m, 2H, arom.), 7.31-7.19 (m, 2H, arom.), 4.42 (dd, J=4.2, 8.3, 1H, CH), 3.41 (dd, J=4.2, 14.9, 1H, CH₂), 3.23 (dd, J=8.3, 14.9, 1H, CH₂).
¹⁹F NMR (400MHz, MeOD) ∂[ppm] = -112.11-(-112.23) (m, 1F).

### c) (Ethylsulfonylsulfanyl)cysteine [2-amino-3-ethylsulfonylsulfanyl-propanoic acid]

An ice-cold solution of 5.93 g sodiumnitrite in 45 ml water was slowly added at 0°C to a stirred solution of 13.57 g cysteine hydrochloride in 90 ml 2 M HCl. Stirring was continued for 1 h at 0°C. Then, 20 g sodium ethanesulfinate was added to the obtained deep red solution and stirring was continued for 2 h at 0°C. Then, additional 5 g sodium ethanesulfinate was added, and stirring of the reaction mixture was continued overnight at 0°C. The resulting pale pink precipitate was filtered off and washed with ice-cold water (2x 10 ml) and diethyl ether (2x 10 ml). The crude product was redissolved in water and neutralized with 2 M NaOH. The product precipitated at a pH in the range of 3-4 and, after complete precipitation, was washed with ice-cold water (2x 10 ml) and diethyl ether (2x 10 ml). Drying *in vacuo* at 21 °C yielded 5.03 g pure (ethylsulfonylsulfanyl)cysteine.
¹H-NMR: (400 MHz, DMSO-d₆) δ [ppm] = 3.50 (m, 4H), 3.32 (m, 1H), 1.28 (t, 3H).

### 2) Preparation of thiol-protected amino acid derivatives of formula (I)

Preparations of N-carboxyanhydrides (NCAs) of formula (I) starting from up to 50 g amino acid of formula (I) were tested without upscaling problems. The amino acid was suspended in 50-300 ml absolute tetrahydrofuran (THF). The suspension was heated to 70°C under reflux and nitrogen atmosphere. The amount of THF used was chosen depending on the scale of the reaction (100 mg amino acid in 50 ml THF, 20 g amino acid in 300 ml THF). 300 ml THF per 50 g amino acid were sufficient to obtain a clear solution of the product (i.e. the NCA). After the addition of diphosgene an intermediate precipitation of amino acid hydrochloride was observed in some cases, leading to a gelation which disappeared after short periods of heating at 70°C. The amount of diphosgene added was in the range of about 0.75-0.8 equivalents (which corresponds to about 1.5-1.6 equivalents of phosgene) at the laboratory scale used. (Higher scales might require less excess of phosgene to complete the reaction within reasonable times.) The reaction was allowed to complete as observable by clarification of the reaction suspension due to the insoluble amino acid being converted into the soluble NCA. Reaction was completed within about 1-3 h after diphosgene addition. The solution was acidified by the release of HCl originating from the phosgene formed during the reaction. To remove said HCl and excess phosgene, dry nitrogen was blown throw the solution for 2-3 h. Thus, the majority of gaseous impurities was removed. Then, high vacuum was applied to ensure remove residual HCl and phosgene. During the high vacuum step, most of the solvent was removed. The product was further purified by performing three consecutive dissolution-precipitation steps under inert gas atmosphere using the solvent THF and precipitating the product with an 1-10 fold excess of the non-solvent hexane. Thus, a colorless to brownish product was obtained. High amounts of the NCA were precipitated slowly, while thin solutions were precipitated at high non-solvent excesses. Ultra sound was used to obtain small crystallites of the product, thus the amount of encapsulated solvent after filtration was low.

### a) ((2-Fluorophenyl)disulfanyl)cysteine-NCA [4-(((2-fluorophenyl)disulfanyl)methyl)oxazolidine-2,5-one]

0.99 g 2-Fluoro-thiophenylcysteine were dispersed in 30 ml absolute THF and heated to 70°C. 0.2 ml Diphosgene were added and the solution turned clear. After 30 min, 0.1 ml diphosgene were added (resulting in a total amount of -2.47 mmol disphosgene) and the solution was stirred for 1.5 h at 70°C. Nitrogen was blown through the solution to remove excess diphosgene and HCl. THF was removed *in vacuo.* The residue was dissolved in 7 ml ethylacetate. The product was precipitated by adding 100 ml hexane. The precipitate was collected by filtration (in the absence of air). The first crop of brown crystals was discarded. The colorless crystals were collected and purified twice by dissolution/precipitation using the solvent ethylacetate and the non-solvent hexane, thus yielding 332 mg ((2-Fluorophenyl)-disulfanyl)cysteine-NCA.
¹H NMR (400MHz, DMSO) ∂[ppm] = 9.31 (s,1H, NH), 7.73 (dt, J=1.8, 7.8, 1H, arom.), 7.48-7.41 (m, 2H, arom.), 7.37-7.27 (m, 2H, arom.), 4.82 (ddd, J=1.3, 4.6, 6.0, 1H, CH), 3.31-3.20 (m, 2H, CH₂).
¹⁹F NMR (400MHz, DMSO) ∂[ppm] = -111.70-(-112.23) (m, 1F).

### b) (Ethylsulfonylsulfanyl)cysteine-NCA [4-(ethylsulfonylsulfanylmethyl)oxazolidine-2,5-one]

3.00 g (ethylsulfonylsulfanyl)cysteine was suspended in 60 ml dry ethyl acetate and heated to 70°C. 3.38 ml diphosgene were added in 3 steps with intervals of 20 min. Then, the suspension was stirred for 4 h at 70°C. Nitrogen was blown through the suspension to remove excess diphosgene, HCl and most of the solvent. The residue was dissolved in 30 ml dry ethyl acetate. Insoluble components were removed by filtration (in the absence air). The product was precipitated by adding 150 ml dry hexane. The precipitate was collected and purified twice by dissolution/precipitation using the solvent ethylacetate and the non-solvent hexane, thus yielding 1.21 g (ethylsulfonylsulfanyl)cysteine-NCA as a pale yellow, crystalline powder.
¹H-NMR: (400 MHz, DMSO-d6) δ [ppm] = 9.33 (s, 1H), 4.85 (s 1H), 3.61 (m, 4H) 1.29z (t, 3H). ¹³C-NMR: (400 MHz, DMSO-d6) δ [ppm] = 173.08, 155.19, 60.50, 59.58, 41.10, 11.74.

### 3) Preparation of block copolymers

Block copolymers were prepared by ring-opening polymerization (ROP) starting from amino acid derivatives of formula (I) (NCAs)
Polymerizations were performed in absolute dimethylformamide (DMF) at temperatures between 5-40°C. Scales between milligrams to several grams were used. The NCA was dissolved in DMF to a concentration in the range of 50-500 mg/ml. For low molecular weight polymers higher concentrations (up to 500 mg/ml) were applied. For high molecular weight polymers lower concentrations (50-100 mg/ml) were applied. After complete dissolution of the NCA the initiator (e.g. neopentylamine) was added. Completion of the reaction was monitored by IR spectroscopy and indicated by the reduction of the NCA peaks intensity (NCA peaks were at 1853 cm⁻¹ and 1786 cm⁻¹). Reaction times varied between hours and days, depending on monomer, reaction temperature, concentrations and chain lengths. Reaction temperature varied between 5-30°C. During polymerization a constant pressure of dry nitrogen was applied to hinder impurities from entering the reaction, while allowing release of the carbon dioxide formed during polymerization. Directly after completion of the reaction the polymer was precipitated to cold ether and centrifuged. DMF was removed by subsequent resuspension in ether in a sonic bath and repeated centrifugation. The precipitate was then dried in high vacuum or lyophilized, if soluble in water or dioxane.

Different block-copolymers were synthesized according to the reaction scheme below. 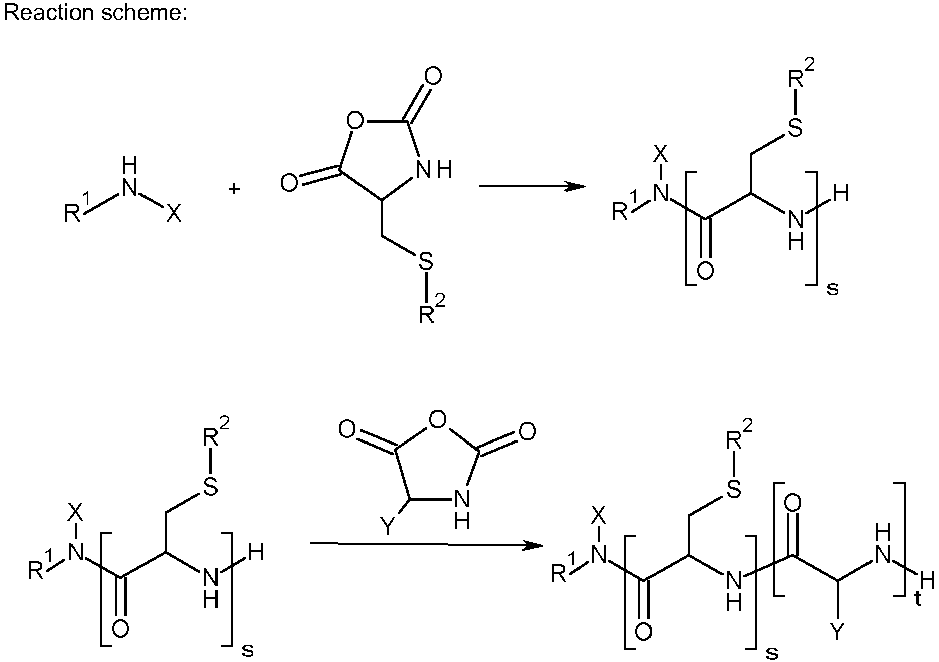
X: H or polymer block (e.g. PEG or polysarcosine)
R¹: H, C₁-C₄-alkyl (e.g. methyl), C₂-C₄-alkenyl (e.g. but-3-enyl) or C₂-C₄-alkynyl (e.g. propargyl)
R²: thiol-protective group as described herein
Y: amino acid residue of a polypeptide block

The compositions and dispersities of three of the prepared block-copolymers are shown in Table 1.

**Table 1: Thiol-protected polysarcosine-block-polycysteine-copolymers**

| example | number of monomeric units in polysarcosine block | number of monomeric units in thiol-protected polycysteine block | number average molecular weight Mn [kg/mol] | dispersity |
|---|---|---|---|---|
| 3a | 200 | 7 | 38.3 | 1.1 |
| 3b | 200 | 13 | 40.2 | 1.1 |
| 3c | 200 | 24 | 42.1 | 1.1 |

### 4) Preparation of core-shell particles and stabilization via cross-linking

a) 15 mg (1eq) block copolymer prepared in either of examples 3a-3c were dissolved in 0.8 ml DMSO. The solution was filtered through a syringe filter (PVDF, 450nm) and the filter was rinsed with 0.4 ml DMSO. The cross-linker hexane-1,6-dithiol (0.6 eq) was added in 0.3 ml DMSO yielding a final concentration of 10 mg/ml. After 30 min, 8 ml water were added to the solution at a rate of 0.5 ml / 15 min and the solution was then dialyzed against water. The final concentration was determined by lyophilizing an aliquot (3 ml) of the final solution and determining the polymer content. Prior to the lightscattering experiment, the solution was filtered, first through a 1 µm syringe filter (Pall) and the through a Millex-HV filter to remove any remaining dust particles.
b) 6.17 mg block copolymer prepared in either of examples 3a-3c were dissolved in 1.4 ml DMSO. 8 ml of acetonitrile were added slowly via a syringe pump over a time of 8 h, resulting in a suspension of self-assembled core-shell particles. 113 µl hexane-1,6-dithiol was mixed with 290 µl triethylamine, 508 µl acetonitrile and 89 µl DMSO to obtain a cross-linker stock solution. 8.67 µl of this cross-linker stock solution were added to 2.6 ml of the suspension of core-shell particles (-0.5 eq. cross-linker / reactive cysteine). The mixture was incubated for 1 h at room temperature. Then, another 8.67 µl of the cross-linker stock solution was added to ensure a complete reaction.

Block copolymers were cross-linked according to the reaction scheme below. 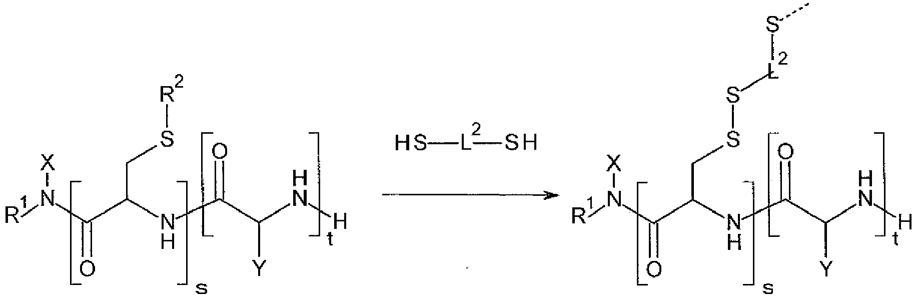
L² = hexenyl, -- = disulfide bond to further thiol group of block copolymer
X, Y, R¹-R² see above.

Further, E1-E45 are described herein:
E1. A thiol-protected amino acid derivative of formula (I) or formula (II) 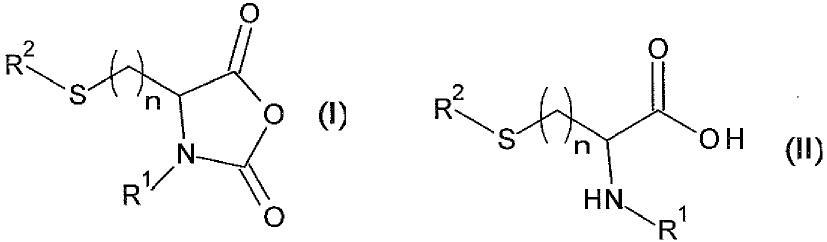 wherein
   n is 1 or 2;
   R¹ is H, C₁-C₄-alkyl, C₂-C₄-alkenyl or C₂-C₄-alkynyl; and
   R² is a group of formula (A) or formula (B) 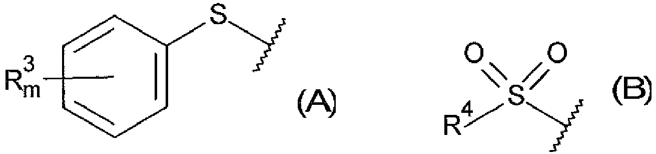 wherein
      m is 1, 2, 3, 4 or 5;
      each R³ is independently selected from halogen, nitro, cyano and C₁-C₄-alkanoyl; and R⁴ is selected from R^{a}-C₁-C₁₆-alkyl, R^{a}, C₁-C₁₆-alkyl, C₂-C₁₆-alkenyl and C₂-C₁₆-alkynyl, wherein the C₁-C₁₆-alkyl, C₂-C₁₆-alkenyl or C₂-C₁₆-alkynyl are unsubstituted or carry 1, 2 or 3 substituents R^{b};
   R^{b} is independently selected from halogeno, cyano, nitro, hydroxyl and thiol;
   R^{a} is selected from:
      (i) phenyl,
      (ii) 5- or 6-membered heteroaromatic monocyclic radicals having 1, 2, 3 or 4 heteroatoms as ring members which are independently selected from O, S and N,
      (iii) 8- to 10-membered heteroaromatic bicarbocyclic radicals having 1, 2, 3 or 4 heteroatoms as ring members which are independently selected from O, S and N, and
      (iv) 8- to 10-membered aromatic bicarbocyclic radicals,
      wherein said radicals (i) to (iv) are unsubstituted or carry 1, 2, 3 or 4 substituents R^{c}, and
   R^{c} is independently selected from halogeno, cyano, nitro, hydroxyl, C₁-C₄-alkyl, C₁-C₄-alkoxy, C₂-C₄-alkenyl, C₂-C₄-alkynyl and C₁-C₄-alkanoyl.
E2. The amino acid derivative of E1 having formula (I) 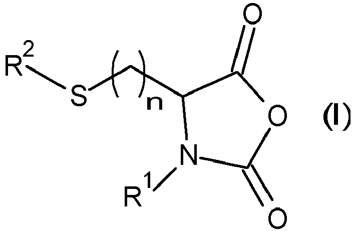
   wherein n and R¹ are defined as in E1 and
   R² is a group of formula (A) 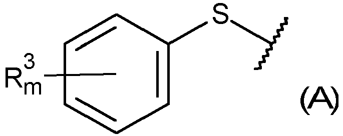 wherein m and R³ are defined as in E1.
E3. The amino acid derivative of E1 having formula (II) 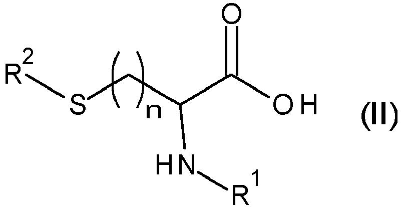
   wherein n and R¹ are defined as in E1 and
   R² is a group of formula (A) 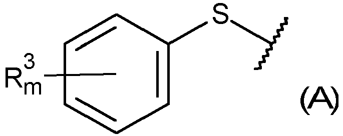 wherein m and R³ are defined as in E1.
E4. The amino acid derivative of E2 or E3, wherein each R³ is independently selected from fluoro, chloro and bromo.
E5. The amino acid derivative of E2 or E3, wherein
   m is 5; and
   R³ is chloro.
E6. The amino acid derivative of E2 or E3, wherein
   m is 1, 2 or 3; and
   R³ is fluoro.
E7. The amino acid derivative of any one of E1-E3, wherein R² is independently selected from groups of formulae (A-1), (A-2), (A-3), (A-4) and (A-5) 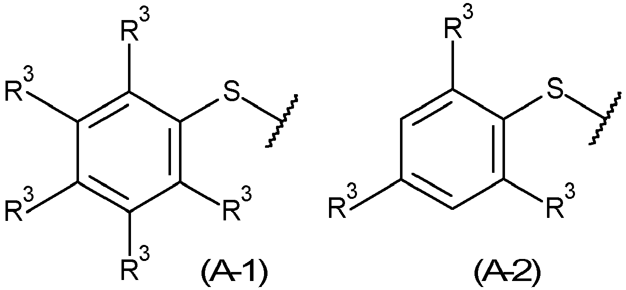 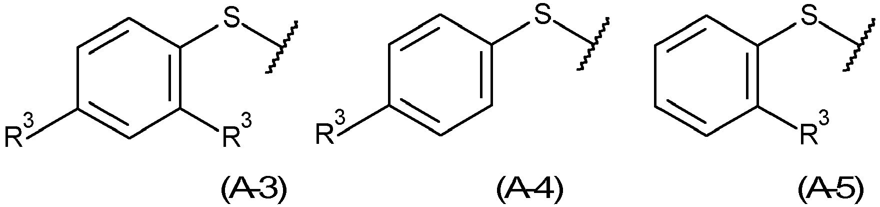 wherein R³ is defined as in any one of E1-E3.
E8. The amino acid derivative of E7, wherein
   R³ is fluoro.
E9. The amino acid derivative of E1 having formula (I) 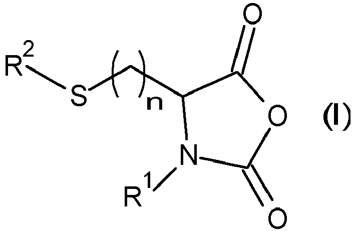
   wherein n and R¹ are defined as in E1 and
   R² is a group of formula (B) 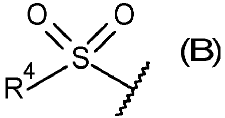 wherein R⁴ is defined as in E1.
E10. The amino acid derivative of E1 having formula (II) 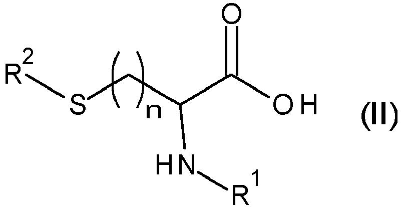
   wherein n and R¹ are defined as in E1 and
   R² is a group of formula (B) 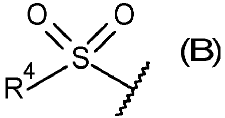 wherein R⁴ is defined as in E1.
E11. The amino acid derivative of E9 or E10, wherein R⁴ is selected from C₁-C₁₆-alkyl, R^{a}-C₁-C₁₆-alkyl and R^{a}, wherein R^{a} is defined as in E1.
E12. The amino acid derivative of E9 or E10, wherein R⁴ is selected from ethyl, butyl, hexyl and benzyl, wherein the benzyl is unsubstituted or carries 1, 2, 3 or 4 substituents R^{c}, and
   R^{c} is defined as in E1.
E13. The amino acid derivative of E9 or E10, wherein R⁴ is selected from unsubstituted C₂-C₁₆-alkenyl and partly or completely halogenated C₂-C₁₆-alkenyl.
E14. A method for preparing a peptide comprising at least one amino acid residue that carries an, optionally protected, thiol-group; the method comprising reacting a thiol-protected amino acid derivative of any one of E1-E13 with an amino acid or with an amino acid residue of a precursor peptide under coupling conditions, thus obtaining a peptide comprising at least one thiol-protected amino acid residue.
E15. The method of E14, wherein the thiol-protected amino acid derivative is reacted with a terminal amino acid residue of the precursor peptide.
E16. The method of E14 or E15, additionally comprising reacting at least part of the thiol-protected amino acid residues of the peptide with a thiol compound, thus obtaining disulfide adducts of said amino acid residues and the thiol compound.
E17. The method of E16, wherein the thiol compound comprises one, two or more than two thiol groups.
E18. The method of any one of E14-E17, wherein the amino acid or the precursor peptide is stably associated with the surface of a polymeric support material.
E19. A method comprising:
   (a) providing a first thiol compound having at least one functional group other than a thiol group, wherein at least one thiol group of said first thiol compound is protected by a group of formula (A) or formula (B) 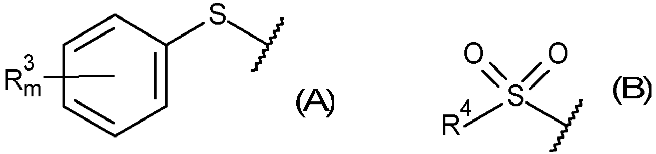 wherein m, R³ and R⁴ are defined as in any one of E1, E4-E6, E8 and E11-E13, so as to form a disulfide with the group of formula (A) or a thiolsulfonate with the group of formula (B);
   (b) effecting a chemical reaction on at least one functional group of the thiol-protected compound produced in step (i) under conditions to which the protected thiol group is chemically inert but to which an unprotected thiol group would be chemically reactive; and
   (c) subsequently either reacting at least part of the protected thiol groups with the thiol group(s) of a second thiol compound, thus obtaining a disulfide compound, or deprotecting the protected thiol group(s).
E20. The method of E19, wherein the thiol group(s) of said first thiol compound are protected by groups of formula (A) 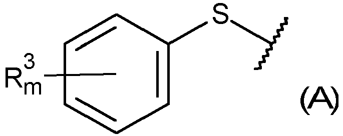 wherein m and R³ are defined as E19.
E21. The method of E20, wherein said first thiol compound comprising at least one thiol group protected by a group of formula (A) is obtained by reacting the free thiol group with a compound of formula (III)

   A-R⁷ **(III)**

   wherein R⁷ is chloro and A is a group of formula (A) defined as in E19.
E22. The method of any one of E19-E21, wherein the group of formula (A) is selected from groups of formulae (A-1), (A-2), (A-3), (A-4) and (A-5) 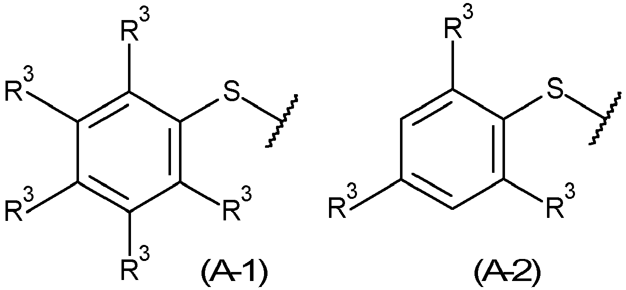 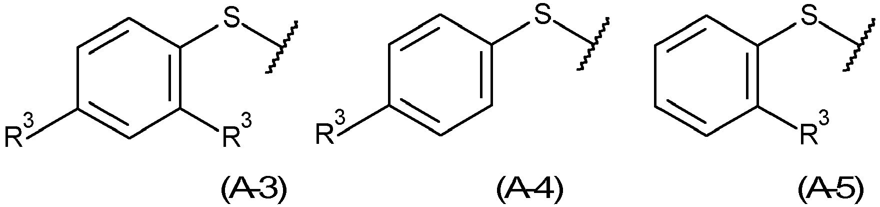 wherein R³ is defined as in E19.
E23. The method of E19, wherein the thiol group(s) of said first thiol compound are protected by groups of formula (B) 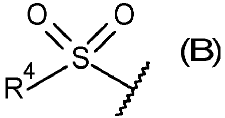 wherein R⁴ is defined as in E19.
E24. The method of any one of E19-E23, wherein the first thiol compound is a thiol-protected amino acid derivative of formula (IV) 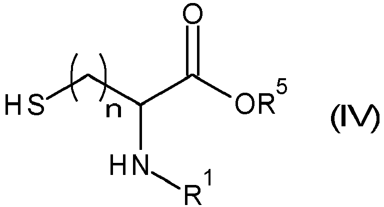 wherein
   n and R¹ are defined as in E1; and
   R⁵ is H, C₁-C₄-alkoxy or a carboxy-protecting group.
E25. The method of any one of E19-E24, wherein the first thiol compound is a peptide comprising at least one amino acid residue of formula (C) 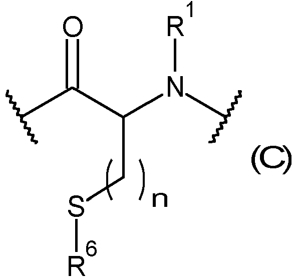 wherein
   n and R¹ are defined as in E1; and
   R⁶ is independently selected from H, a group of formula (A) and a group of formula (B) 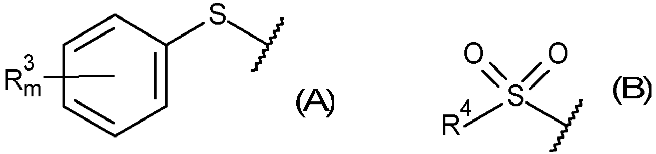 wherein m, R³ and R⁴ are defined as in any one of E1, E4-E6, E8 and E11-E13.
E26. A polymer comprising a thiol-reactive block, wherein
   the thiol-reactive block is a linear polymer of 1-1000 monomeric units of formula (C) 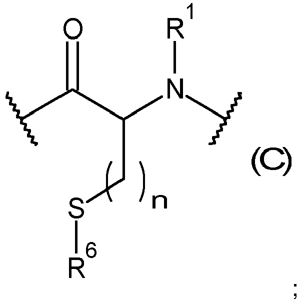
   n and R¹ are defined as in E1; and
   R⁶ is independently selected from H, a group of formula (A) and a group of formula (B) 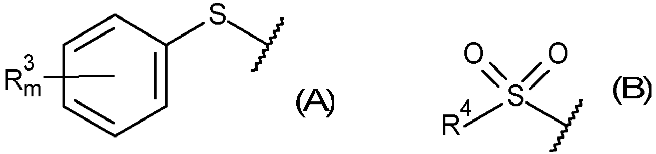 wherein m, R³ and R⁴ are defined as in any one of E1, E4-E6, E8 and E11-E13.
E27. The polymer of E26, wherein the polymer is a block copolymer further comprising a hydrophilic block, wherein the hydrophilic block is a linear polymer of 10-1000 monomeric units.
E28. The block copolymer of E27 which is a compound of formula (V) 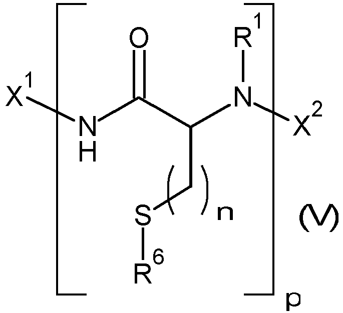 wherein
   X¹ is said hydrophilic block;
   p is 1-1000;
   n, R¹ and R⁶ are defined as in E26; and
   X² is H or at least one further block of the block copolymer.
E29. The polymer of any one of E26-E28, wherein in at least one monomeric unit of formula (C) or formula (V) R⁶ is a group of formula (A) 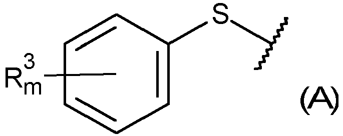 wherein m and R³ are defined as in E26.
E30. The polymer of E29, wherein the group of formula (A) is independently selected from groups of formulae (A-1), (A-2), (A-3), (A-4) and (A-5) 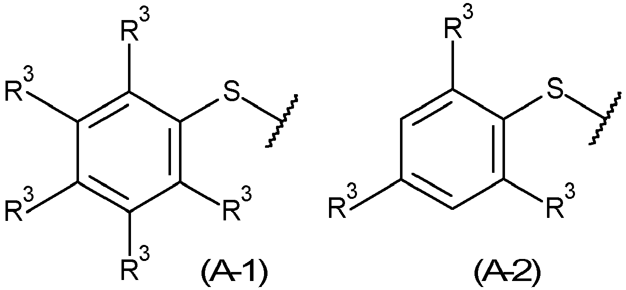 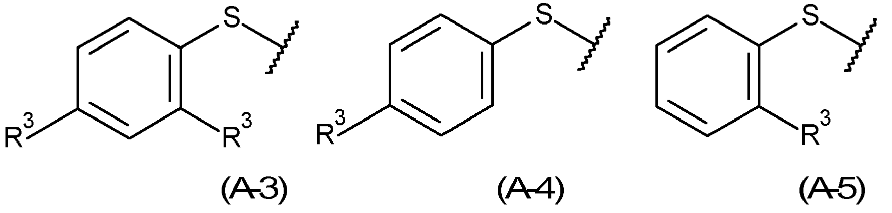 wherein R³ is defined as in E26.
E31. The polymer of any one of E26-E28, wherein in at least one monomeric unit of formula (C) R⁶ is a group of formula (B) 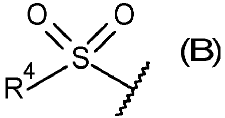 wherein R⁴ is defined as in E26.
E32. The block copolymer of any one of E27-E31, wherein the hydrophilic block is independently selected from hydrophilic polypeptide moieties, poly(ethylene) glycol moieties, hydrophilic polysaccharide moieties, optionally substituted polymethacrylic acid moieties, optionally substituted polyacrylic acid moieties, polyvinylalcohol moieties, polyvinylpyrollidone moieties and poly(oxazoline) moieties.
E33. The block copolymer of E32, wherein the hydrophilic block is polysarcosine.
E34. The block copolymer of any one of E27-E33, further comprising a polycationic block of 1-1000 monomeric units.
E35. The block copolymer of E34, wherein the monomeric units of the polycationic block are units of formula (D) 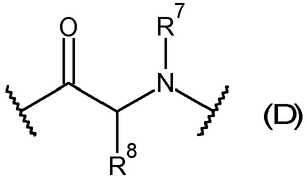 wherein
   R⁷ is H or C₁-C₄-alkyl; and
   R⁸ is independently selected from 4-aminobutyl, 3-guanidinopropyl and 1H-imidazol-4-ylmethyl.
E36. A method for preparing a block copolymer comprising:
   (iii) providing a hydrophilic polymer of 10-1000 monomeric units that carries at least one terminal primary or secondary amino group; and
   (iv) reacting a thiol-protected amino acid derivative of formula (I) or formula (II) defined as in any one of E1-E13 with the terminal amino group of the hydrophilic polymer,
   thus forming a polymer that carries a terminal primary or secondary amino group.
E37. The method of E36 further comprising:
   (v) reacting a thiol-protected amino acid derivative of formula (I) or formula (II) defined as in any one of E1-E13 with the terminal amino group of the polymer obtained in step (ii); and
   (vi) optionally repeating the reacting of a thiol-protected amino acid derivative of formula (I) with the obtained polymer once or more than once.
E38. A core-shell particle comprising:
   a multitude of block copolymers according to any one of E26-E35, and
   at least one cargo molecule, e.g. a pharmaceutically active agent or a polynucleotide,
   encapsulated therein;
   wherein the hydrophilic blocks of the block copolymers are located in the shell region of the particle, and the thiol-reactive blocks of the block copolymers as well as the cargo molecule(s) are located in the core region of the particle.
E39. The core-shell particle of E38, wherein
   at least part of the cargo molecule(s) is covalently bound to the thiol-reactive blocks of the block copolymers.
E40. The core-shell particle of E39, wherein
   the covalently bound cargo molecule and a monomer of the thiol-reactive block form the structure (E) 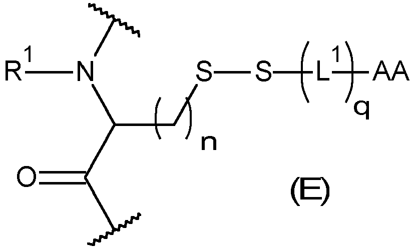 wherein
   n and R¹ are defined as in E1;
   q is 0 or 1;
   L¹ is a linker; and
   AA is the cargo molecule.
E41. A core-shell particle as defined in any one of E38-E40, wherein at least two monomers of the thiol-reactive blocks of the block copolymers are cross-linked via a cross-linker comprising two or more than two thiol groups.
E42. The core-shell particle of E41, wherein
   the at least two monomers of the thiol-reactive blocks cross-linked via the cross-linker form the structure (F) 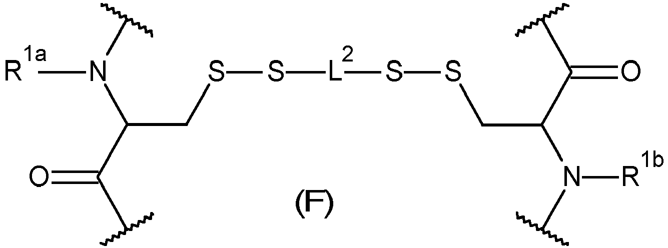 wherein
   R^{1a} and R^{1b} are independently selected from groups R¹ defined as in E1; and
   -S-L²-S- is the cross-linker residue.
E43. The core-shell particle of E41 or E42, wherein
   at least part of the pharmaceutically active agent is covalently bound to the cross-linker residue -S-L²-S-.
E44. A method for stabilizing a core-shell particle according to any one of E38-E40, the method comprising contacting the core-shell particle with a cross-linker comprising two or more than two thiol groups.
E45. A composition comprising the core-shell particle according to any one of E38-E43 and a pharmaceutically acceptable carrier.

## Claims

1. A method for preparing a peptide comprising at least one amino acid residue that carries an, optionally protected, thiol-group; the method comprising reacting a thiol-protected amino acid derivative with an amino acid or with an amino acid residue of a precursor peptide under coupling conditions, thus obtaining a peptide comprising at least one thiol-protected amino acid residue wherein the thiol-protected amino acid derivative is a compound of formula (I) or formula (II) 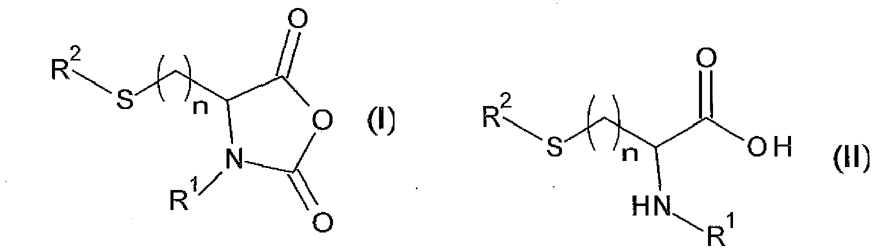 wherein
n is 1 or 2;
R¹ is H, C₁-C₄-alkyl, C₂-C₄-alkenyl or C₂-C₄-alkynyl; and
R² is a group of formula (A) or formula (B) 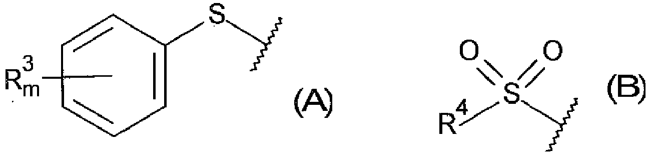 wherein
m is 1, 2, 3, 4 or 5, and each R³ is independently selected from halogen, nitro, cyano and C₁-C₄-alkanoyl;
preferably m is 1, 2, 3, 4 or 5, and each R³ is independently selected from fluoro, chloro and bromo;
more preferably m is 5, and R³ is chloro, or m is 1, 2 or 3, and R³ is fluoro;
R⁴ is selected from R^{a}-C₁-C₁₆-alkyl, R^{a}, C₁-C₁₆-alkyl, C₂-C₁₆-alkenyl and C₂-C₁₆-alkynyl, wherein the C₁-C₁₆-alkyl, C₂-C₁₆-alkenyl or C₂-C₁₆-alkynyl are unsubstituted or carry 1, 2 or 3 substituents R^{b};
preferably R⁴ is selected from C₁-C₁₆-alkyl, R^{a}-C₁-C₁₆-alkyl, R^{a}, unsubstituted C₂-C₁₆-alkenyl and partly or completely halogenated C₂-C₁₆-alkenyl;
more preferably R⁴ is selected from ethyl, butyl, hexyl and benzyl, wherein the benzyl is unsubstituted or carries 1, 2, 3 or 4 substituents R^{c};
R^{b} is independently selected from halogeno, cyano, nitro, hydroxyl and thiol;
R^{a} is selected from:
(i) phenyl,
(ii) 5- or 6-membered heteroaromatic monocyclic radicals having 1, 2, 3 or 4 heteroatoms as ring members which are independently selected from O, S and N,
(iii) 8- to 10-membered heteroaromatic bicarbocyclic radicals having 1, 2, 3 or 4 heteroatoms as ring members which are independently selected from O, S and N, and
(iv) 8- to 10-membered aromatic bicarbocyclic radicals,
wherein said radicals (i) to (iv) are unsubstituted or carry 1, 2, 3 or 4 substituents R^{c}; and
R^{c} is independently selected from halogeno, cyano, nitro, hydroxyl, C₁-C₄-alkyl, C₁-C₄-alkoxy, C₂-C₄-alkenyl, C₂-C₄-alkynyl and C₁-C₄-alkanoyl.

2. A polymer comprising a thiol-reactive block, wherein
the thiol-reactive block consists of 1-1000 monomeric units of formula (C) 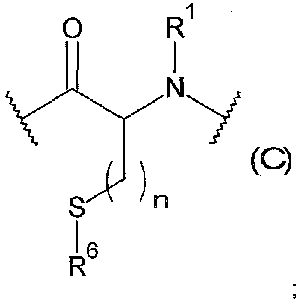 n and R¹ are defined as in claim 1; and
R⁶ is independently selected from H, a group of formula (A) and a group of formula (B) 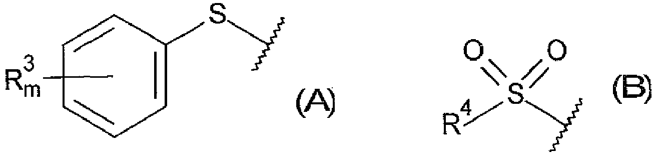 wherein m, R³ and R⁴ are defined as in claim 1, and wherein in at least one monomeric unit of formula (C) R⁶ is a group of formula (A) or formula (B).

3. A method for preparing a block copolymer comprising:
(i) providing a hydrophilic polymer of 10-1000 monomeric units that carries at least one terminal primary or secondary amino group; and
(ii) reacting a thiol-protected amino acid derivative of formula (I) or formula (II) defined as in claim 1 with the terminal amino group of the hydrophilic polymer,
thus forming a polymer that carries a terminal primary or secondary amino group.

4. A core-shell particle comprising:
a multitude of block copolymers, and
at least one cargo molecule, e.g. a pharmaceutically active agent or a polynucleotide, encapsulated therein;
wherein each of the block copolymers comprises a thiol-reactive block as defined in claim 2 and a hydrophilic block that is a linear polymer of 10-1000 monomeric units;
the hydrophilic blocks of the block copolymers are located in the shell region of the particle, and the thiol-reactive blocks of the block copolymers as well as the cargo molecule(s) are located in the core region of the particle.

5. A method for stabilizing a core-shell particle according to claim 4, the method comprising contacting the core-shell particle with a cross-linker comprising two or more than two thiol groups.

6. A composition comprising the core-shell particle according to claim 4 and a pharmaceutically acceptable carrier.

7. A thiol-protected amino acid derivative of formula (I) or formula (II) 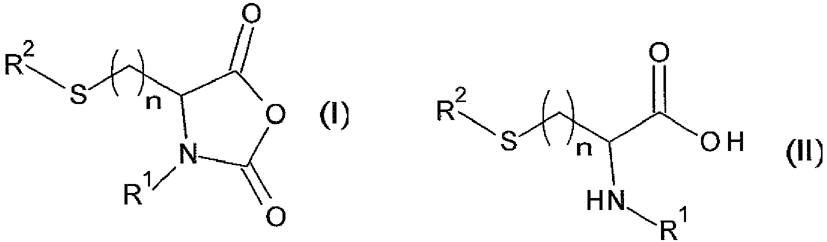 wherein
n is 1 or 2;
R¹ is H, C₁-C₄-alkyl, C₂-C₄-alkenyl or C₂-C₄-alkynyl; and
R² is a group of formula (A) or formula (B) 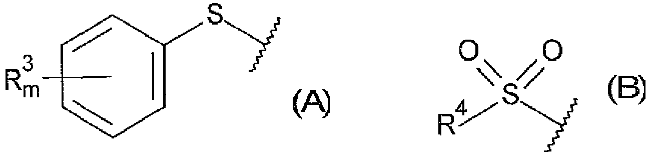 wherein
m is 1, 2, 3, 4 or 5;
each R³ is independently selected from halogen, cyano and C₁-C₄-alkanoyl; and
R⁴ is selected from R^{a}-C₁-C₁₆-alkyl, ethyl, n-propyl, isopropyl, n-butyl, 1-methylpropyl (sec-butyl), 2-methylpropyl (isobutyl) and 1,1-dimethylethyl (tert-butyl), n-pentyl, 1-methylbutyl, 2-methylbutyl, 3-methylbutyl (isopentyl), 2,2-dimethylpropyl, 1-ethylpropyl, 1,1-dimethylpropyl, 1,2-dimethylpropyl, n-hexyl, 1-methylpentyl, 2-methylpentyl, 3-methylpentyl, 4-methylpentyl (isohexyl), 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, n-octyl, 2-methylheptyl, 1-ethylhexyl, 2-ethylhexyl, 1,2-dimethylhexyl, 1-propylpentyl, 2-propylpentyl, C₂-C₁₆-alkenyl and C₂-C₁₆-alkynyl, wherein the C₁-C₁₆-alkyl, ethyl, n-propyl, isopropyl, n-butyl, 1-methylpropyl (sec-butyl), 2-methylpropyl (isobutyl) and 1,1-dimethylethyl (tert-butyl), n-pentyl, 1-methylbutyl, 2-methylbutyl, 3-methylbutyl (isopentyl), 2,2-dimethylpropyl, 1-ethylpropyl, 1,1-dimethylpropyl, 1,2-dimethylpropyl, n-hexyl, 1-methylpentyl, 2-methylpentyl, 3-methylpentyl, 4-methylpentyl(isohexyl), 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, n-octyl, 2-methylheptyl, 1-ethylhexyl, 2-ethylhexyl, 1,2-dimethylhexyl, 1-propylpentyl, 2-propylpentyl, C₂-C₁₆-alkenyl or C₂-C₁₆-alkynyl are unsubstituted or carry 1, 2 or 3 substituents R^{b};
R^{b} is independently selected from halogeno, cyano, nitro, hydroxyl and thiol;
R^{a} is selected from:
(i) phenyl,
(ii) 5- or 6-membered heteroaromatic monocyclic radicals having 1, 2, 3 or 4 heteroatoms as ring members which are independently selected from O, S and N,
(iii) 8- to 10-membered heteroaromatic bicarbocyclic radicals having 1, 2, 3 or 4 heteroatoms as ring members which are independently selected from O, S and N, and
(iv) 8- to 10-membered aromatic bicarbocyclic radicals,
wherein said radicals (i) to (iv) are unsubstituted or carry 1, 2, 3 or 4 substituents R^{c}, and
R^{c} is independently selected from halogeno, cyano, nitro, hydroxyl, C₁-C₄-alkyl, C₁-C₄-alkoxy, C₂-C₄-alkenyl, C₂-C₄-alkynyl and C₁-C₄-alkanoyl.

8. The amino acid derivative of claim 7,
wherein R² is a group of formula (A) 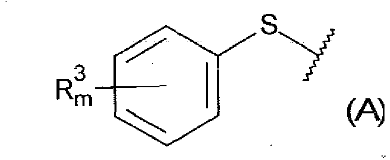 wherein m and R³ are defined as in claim 7.

9. The amino acid derivative of claim 8, wherein each R³ is independently selected from fluoro, chloro and bromo.

10. The amino acid derivative of claim 8, wherein
m is 5, and R³ is chloro; or
m is 1, 2 or 3, and R³ is fluoro.

11. The amino acid derivative of claim 7,
wherein R² is a group of formula (B) 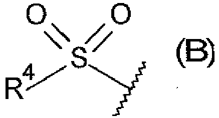 wherein R⁴ is defined as in claim 7.

12. The amino acid derivative of claim 11, wherein R⁴ is selected from ethyl, n-propyi, isopropyl, n-butyl, 1-methylpropyl(sec-butyl), 2-methylpropyl(isobutyl) and 1,1-dimethylethyl (tert-butyl), n-pentyl, 1-methylbutyl, 2-methylbutyl, 3-methylbutyl (isopentyl), 2,2-dimethylpropyl, 1-ethylpropyl, 1,1-dimethylpropyl, 1,2-dimethylpropyl, n-hexyl, 1-methylpentyl, 2-methylpentyl, 3-methylpentyl, 4-methylpentyl (isohexyl), 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-heptyl, 1-methylhexyl, 2-methylhexyl, 3-methylhexyl, 4-methylhexyl, 5-methylhexyl, 1-ethylpentyl, 2-ethylpentyl, 3-ethylpentyl, n-octyl, 2-methylheptyl, 1-ethylhexyl, 2-ethylhexyl, 1,2-dimethylhexyl, 1-propylpentyl, 2-propylpentyl and R^{a}-C₁-C₁₆-alkyl, wherein R^{a} is defined as in claim 7.

13. The amino acid derivative of claim 11, wherein R⁴ is selected from ethyl, butyl, hexyl and benzyl, wherein the benzyl is unsubstituted or carries 1, 2, 3 or 4 substituents R^{c}, and R^{c} is defined as in claim 7.

14. The amino acid derivative of claim 11, wherein R⁴ is selected from unsubstituted C₂-C₁₆-alkenyl and partly or completely halogenated C₂-C₁₆-alkenyl.

## Patentansprüche

1. Verfahren zur Herstellung eines Peptids, das mindestens einen Aminosäurerest umfasst, welcher eine gegebenenfalls geschützte Thiolgruppe trägt;
wobei man ein thiolgeschütztes Aminosäurederivat mit einer Aminosäure oder mit einem Aminosäurerest eines Vorläufer-Peptids unter Kupplungsbedingungen umsetzt und so ein Peptid erhält, welches mindestens einen thiolgeschützten Aminosäurerest umfasst, wobei das thiolgeschützte Aminosäurederivat eine Verbindung der Formel (I) oder (II) 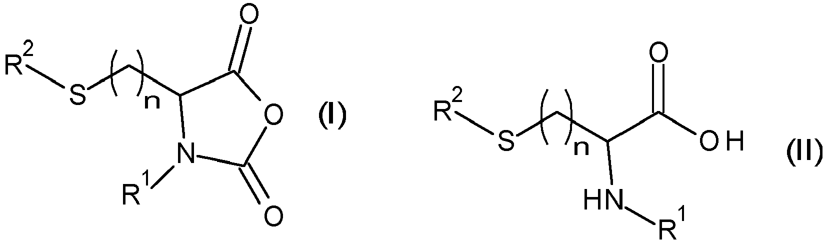 ist, worin
n für 1 oder 2 steht;
R¹ für H, C₁-C₄-Alkyl, C₂-C₄-Alkenyl oder C₂-C₄-Alkinyl steht; und
R² für eine Gruppe der Formel (A) oder Formel (B) 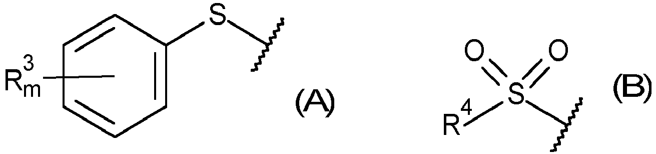 steht, worin
m für 1, 2, 3, 4 oder 5 steht und R³ jeweils unabhängig ausgewählt ist unter Halogen, Nitro, Cyano und C₁-C₄-Alkanoyl;
m vorzugsweise für 1, 2, 3, 4 oder 5 steht und R³ jeweils unabhängig ausgewählt ist unter Fluor, Chlor und Brom;
m besonders bevorzugt für 5 und R³ für Chlor steht, oder m für 1, 2 oder 3 und R³ für Fluor steht;
R⁴ ausgewählt ist unter R^{a}-C₁-C₁₆-Alkyl, R^{a}, C₁-C₁₆-Alkyl, C₂-C₁₆-Alkenyl und C₂-C₁₆-Alkinyl, wobei das C₁-C₁₆-Alkyl, C₂-C₁₆-Alkenyl oder C₂-C₁₆-Alkinyl unsubstituiert sind oder 1, 2 oder 3 Substituenten R^{b} tragen;
R⁴ vorzugsweise ausgewählt ist unter C₁-C₁₆-Alkyl, R^{a}-C₁-C₁₆-Alkyl, R^{a}, unsubstituiertem C₂-C₁₆-Alkenyl und teilweise oder vollständig halogeniertem C₂-C₁₆-Alkenyl;
R⁴ besonders bevorzugt ausgewählt ist unter Ethyl, Butyl, Hexyl und Benzyl, wobei das Benzyl unsubstituiert ist oder 1, 2, 3 oder 4 Substituenten R^{c} aufweist;
R^{b} unabhängig ausgewählt ist unter Halogen, Cyano, Nitro, Hydroxyl und Thiol;
R^{a} ausgewählt ist unter:
(i) Phenyl,
(ii) 5- oder 6-gliedrigen heteroaromatischen monocyclischen Resten mit 1, 2, 3 oder 4 Heteroatomen als Ringglieder, welche unabhängig voneinander ausgewählt sind unter O, S und N,
(iii) 8- bis 10-gliedrigen heteroaromatischen bicarbocyclischen Resten mit 1, 2, 3 oder 4 Heteroatomen als Ringglieder, welche unabhängig voneinander ausgewählt sind unter O, S und N, und
(iv) 8- bis 10-gliedrigen aromatischen bicarbocyclischen Resten,
wobei die Reste (i) bis (iv) unsubstituiert sind oder 1, 2, 3 oder 4 Substituenten R^{c} tragen; und
R^{c} unabhängig ausgewählt ist unter Halogen, Cyano, Nitro, Hydroxyl, C₁-C₄-Alkyl, C₁-C₄-Alkoxy, C₂-C₄-Alkenyl, C₂-C₄-Alkinyl und C₁-C₄-Alkanoyl.

2. Polymer, umfassend einen thiolreaktiven Block, wobei
der thiolreaktive Block aus 1-1000 monomeren Einheiten der Formel (C) 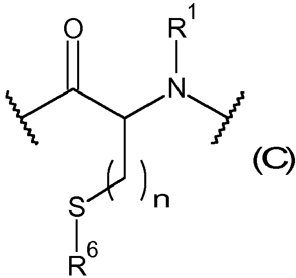 besteht;
n und R¹ wie in Anspruch 1 definiert sind; und
R⁶ unabhängig ausgewählt ist unter H, einer Gruppe der Formel (A) und einer Gruppe der Formel (B) 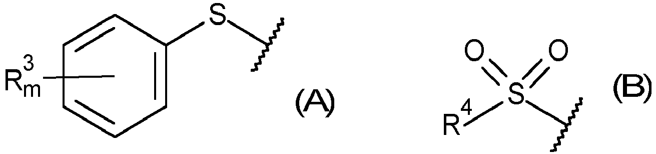 worin m, R³ und R⁴ wie in Anspruch 1 definiert sind, und worin in mindestens einer monomeren Einheit der Formel (C) R⁶ für eine Gruppe der Formel (A) oder Formel (B) steht.

3. Verfahren zur Herstellung eines Blockcopolymers, wobei man:
(i) ein hydrophiles Polymer aus 10-1000 monomeren Einheiten bereitstellt, welches mindestens eine terminale primäre oder sekundäre Aminogruppe aufweist; und
(ii) ein thiolgeschütztes Aminosäurederivat der Formel (I) oder Formel (II) nach Anspruch 1 mit der terminalen Aminogruppe des hydrophilen Polymers umsetzt,
um ein Polymer mit einer terminalen primären oder sekundären Aminogruppe zu bilden.

4. Kern-Schale-Partikel, umfassend:
eine Vielzahl an Blockcopolymeren und
wenigstens ein darin verkapseltes Cargomolekül, beispielsweise einen pharmazeutischen Wirkstoff oder ein Polynucleotid;
wobei die Blockcopolymere jeweils einen thiolreaktiven Block nach Anspruch 2 und einen hydrophilen Block, welcher ein lineares Polymer mit 10-1000 monomeren Einheiten ist, umfassen; die hydrophilen Blöcke der Blockcopolymere im Bereich der Schale des Partikels angeordnet sind, und sowohl die thiolreaktiven Blöcke der Blockcopolymere als auch das/die Cargomolekül/e im Bereich des Kerns des Partikels angeordnet sind.

5. Verfahren zur Stabilisierung eines Kern-Schale-Partikels nach Anspruch 4, wobei man den Kern-Schale-Partikel mit einem zwei oder mehr als zwei Thiolgruppen umfassenden Vernetzer in Kontakt bringt.

6. Zusammensetzung, umfassend den Kern-Schale-Partikel nach Anspruch 4 und einen pharmazeutisch annehmbaren Träger.

7. Thiolgeschütztes Aminosäurederivat der Formel (I) oder Formel (II) 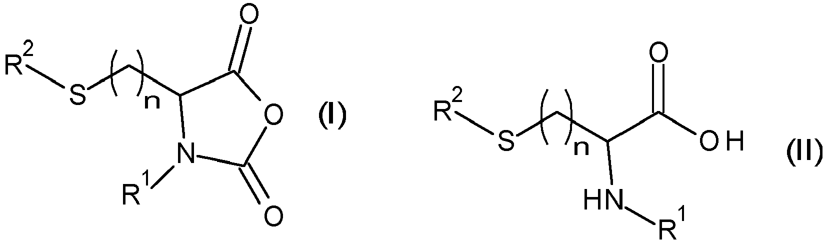 worin
n für 1 oder 2 steht;
R¹ für H, C₁-C₄-Alkyl, C₂-C₄-Alkenyl oder C₂-C₄-Alkinyl steht; und
R² für eine Gruppe der Formel (A) oder Formel (B) 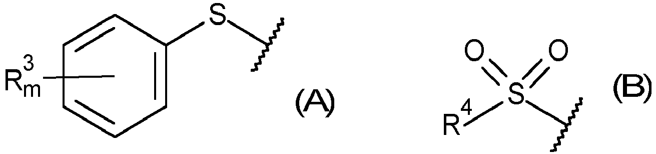 steht, worin
m für 1, 2, 3, 4 oder 5 steht;
R³ jeweils unabhängig ausgewählt ist unter Halogen, Cyano und C₁-C₄-Alkanoyl; und
R⁴ ausgewählt ist unter R^{a}-C₁-C₁₆-Alkyl, Ethyl, n-Propyl, Isopropyl, n-Butyl, 1-Methylpropyl (sec-Butyl), 2-Methylpropyl (Isobutyl) und 1,1-Dimethylethyl (tert-Butyl), n-Pentyl, 1-Methylbutyl, 2-Methylbutyl, 3-Methylbutyl (Isopentyl), 2,2-Dimethylpropyl, 1-Ethylpropyl, 1,1-Dimethylpropyl, 1,2-Dimethylpropyl, n-Hexyl, 1-Methylpentyl, 2-Methylpentyl, 3-Methylpentyl, 4-Methylpentyl (Isohexyl), 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 3-Ethylpentyl, n-Octyl, 2-Methylheptyl, 1-Ethylhexyl, 2-Ethylhexyl, 1,2-Dimethylhexyl, 1-Propylpentyl, 2-Propylpentyl, C₂-C₁₆-Alkenyl und C₂-C₁₆-Alkinyl, wobei das C₁-C₁₆-Alkyl, Ethyl, n-Propyl, Isopropyl, n-Butyl, 1-Methylpropyl (sec-Butyl), 2-Methylpropyl (Isobutyl) und 1,1-Dimethylethyl (tert-Butyl), n-Pentyl, 1-Methylbutyl, 2-Methylbutyl, 3-Methylbutyl (Isopentyl), 2,2-Dimethylpropyl, 1-Ethylpropyl, 1,1-Dimethylpropyl, 1,2-Dimethylpropyl, n-Hexyl, 1-Methylpentyl, 2-Methylpentyl, 3-Methylpentyl, 4-Methylpentyl (Isohexyl), 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 3-Ethylpentyl, n-Octyl, 2-Methylheptyl, 1-Ethylhexyl, 2-Ethylhexyl, 1,2-Dimethylhexyl, 1-Propylpentyl, 2-Propylpentyl, C₂-C₁₆-Alkenyl oder C₂-C₁₆-Alkinyl unsubstituiert sind 1, 2 oder 3 Substituenten R^{b} tragen;
R^{b} unabhängig ausgewählt ist unter Halogen, Cyano, Nitro, Hydroxyl und Thiol;
R^{a} ausgewählt ist unter:
(i) Phenyl,
(ii) 5- oder 6-gliedrigen heteroaromatischen monocyclischen Resten mit 1, 2, 3 oder 4 Heteroatomen als Ringglieder, welche unabhängig voneinander ausgewählt sind unter O, S und N,
(iii) 8- bis 10-gliedrigen heteroaromatischen bicarbocyclischen Resten mit 1, 2, 3 oder 4 Heteroatomen als Ringglieder, welche unabhängig voneinander ausgewählt sind unter O, S und N, und
(iv) 8- bis 10-gliedrigen aromatischen bicarbocyclischen Resten,
wobei die Reste (i) bis (iv) unsubstituiert sind oder 1, 2, 3 oder 4 Substituenten R^{c} tragen; und
R^{c} unabhängig ausgewählt ist unter Halogen, Cyano, Nitro, Hydroxyl, C₁-C₄-Alkyl, C₁-C₄-Alkoxy, C₂-C₄-Alkenyl, C₂-C₄-Alkinyl und C₁-C₄-Alkanoyl.

8. Aminosäurederivat nach Anspruch 7,
wobei R² für eine Gruppe der Formel (A) 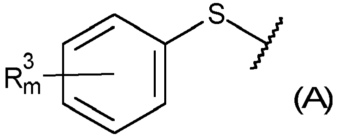 steht, worin m und R³ wie in Anspruch 7 definiert sind.

9. Aminosäurederivat nach Anspruch 8, wobei R³ jeweils unabhängig ausgewählt ist unter Fluor, Chlor und Brom.

10. Aminosäurederivat nach Anspruch 8, wobei
m für 5 und R³ für Chlor steht; oder
m für 1, 2 oder 3 und R³ für Fluor steht.

11. Aminosäurederivat nach Anspruch 7,
wobei R² für eine Gruppe der Formel (B) 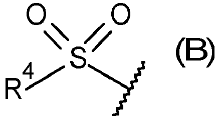 steht, worin R⁴ wie in Anspruch 7 definiert ist.

12. Aminosäurederivat nach Anspruch 11, wobei R⁴ ausgewählt ist unter Ethyl, n-Propyl, Isopropyl, n-Butyl, 1-Methylpropyl (sec-Butyl), 2-Methylpropyl (Isobutyl) und 1,1-Dimethylethyl (tert-Butyl), n-Pentyl, 1-Methylbutyl, 2-Methylbutyl, 3-Methylbutyl (Isopentyl), 2,2-Dimethylpropyl, 1-Ethylpropyl, 1,1-Dimethylpropyl, 1,2-Dimethylpropyl, n-Hexyl, 1-Methylpentyl, 2-Methylpentyl, 3-Methylpentyl, 4-Methylpentyl (Isohexyl), 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 3-Ethylpentyl, n-Octyl, 2-Methylheptyl, 1-Ethylhexyl, 2-Ethylhexyl, 1,2-Dimethylhexyl, 1-Propylpentyl, 2-Propylpentyl und R^{a}-C₁-C₁₆-Alkyl, wobei R^{a} wie in Anspruch 7 definiert ist.

13. Aminosäurederivat nach Anspruch 11, wobei R⁴ ausgewählt ist unter Ethyl, Butyl, Hexyl und Benzyl, wobei das Benzyl unsubstituiert ist und 1, 2, 3 oder 4 Substituenten R^{c} aufweist und R^{c} wie in Anspruch 7 definiert ist.

14. Aminosäurederivat nach Anspruch 11, wobei R⁴ ausgewählt ist unter unsubstituiertem C₂-C₁₆-Alkenyl und teilweise oder vollständig halogeniertem C₂-C₁₆-Alkenyl.

## Revendications

1. Procédé de préparation d'un peptide comprenant au moins un résidu d'acide aminé qui comporte un groupe thiol, facultativement protégé ; le procédé comprenant la réaction d'un dérivé d'acide aminé à thiol protégé avec un acide aminé ou avec un résidu d'acide aminé d'un peptide précurseur dans des conditions de couplage, de façon à obtenir un peptide comprenant au moins un résidu d'acide aminé à thiol protégé, le dérivé d'acide aminé à thiol protégé étant un composé de formule (I) ou de formule (II) 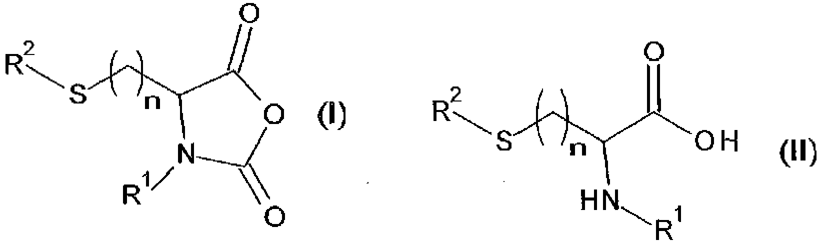 dans lequel
n est 1 ou 2 ;
R¹ est H, alkyle en C₁-C₄, alcényle en C₂-C₄ ou alcynyle en C₂-C₄ ; et
R² est un groupe de formule (A) ou formule (B) 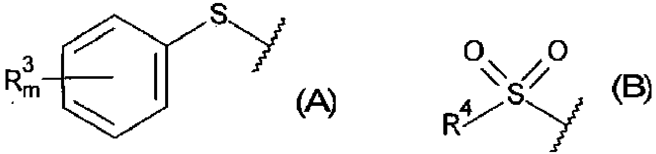 dans lequel
m est 1, 2, 3, 4 ou 5, et chaque R³ est indépendamment choisi parmi halogène, nitro, cyano et alcanoyle en C₁-C₄ ;
de préférence m est 1, 2, 3, 4 ou 5, et chaque R³ est indépendamment choisi parmi fluoro, chloro et bromo ; plus préférablement, m est 5, et R³ est chloro, ou m est 1, 2 ou 3, et R³ est fluoro ;
R⁴ est choisi parmi R^{a}-(alkyle en C₁-C₁₆), R^{a}, alkyle en C₁-C₁₆, alcényle en C₂-C₁₆ et alcynyle en C₂-C₁₆, où les alkyle en C₁-C₁₆, alcényle en C₂-C₁₆ ou alcynyle en C₂-C₁₆ sont non substitués ou comportent 1, 2 ou 3 substituants R^{b} ;
de préférence R⁴ est choisi parmi alkyle en C₁-C₁₆, R^{a}-(alkyle en C₁-C₁₆), R^{a}, alcényle en C₂-C₁₆ non substitué et alcényle en C₂-C₁₆ partiellement ou complètement halogéné ;
plus préférablement, R⁴ est choisi parmi éthyle, butyle, hexyle et benzyle, le benzyle étant non substitué ou comportant 1, 2, 3 ou 4 substituants R^{c} ;
R^{b} est indépendamment choisi parmi halogéno, cyano, nitro, hydroxyle et thiol ;
R^{a} est choisi parmi :
(i) phényle,
(ii) des radicaux monocycliques hétéroaromatiques de 5 ou 6 chaînons ayant 1, 2, 3 ou 4 hétéroatomes en tant que chaînons de cycle qui sont indépendamment choisis parmi 0, S et N,
(iii) des radicaux bicarbocycliques hétéroaromatiques de 8 à 10 chaînons ayant 1, 2, 3 ou 4 hétéroatomes en tant que chaînons de cycle qui sont indépendamment choisis parmi 0, S et N, et
(iv) des radicaux bicarbocycliques aromatiques de 8 à 10 chaînons,
lesdits radicaux (i) à (iv) étant non substitués ou comportant 1, 2, 3 ou 4 substituants R^{c} ; et
R^{c} est indépendamment choisi parmi halogéno, cyano, nitro, hydroxyle, alkyle en C₁-C₄, alcoxy en C₁-C₄, alcényle en C₂-C₄, alcynyle en C₂-C₄ et alcanoyle en C₁-C₄.

2. Polymère comprenant un bloc réactif avec thiol, dans lequel
le bloc réactif avec thiol est constitué de 1 à 1000 motifs monomères de formule (C) 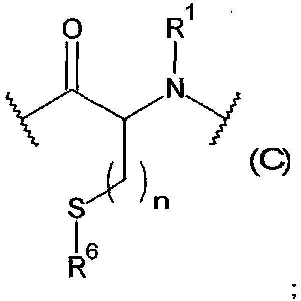
n et R¹ sont tels que définis dans la revendication 1 ; et
R⁶ est indépendamment choisi parmi H, un groupe de formule (A) et un groupe de formule (B) 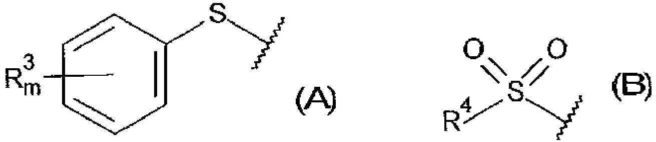 dans lequel m, R³ et R⁴ sont tels que définis dans la revendication 1, et dans lequel, dans au moins un motif monomère de formule (C), R⁶ est un groupe de formule (A) ou formule (B).

3. Procédé de préparation d'un copolymère séquencé comprenant :
(i) la fourniture d'un polymère hydrophile de 10 à 1000 motifs monomères qui comporte au moins un groupe amino primaire ou secondaire terminal ; et
(ii) la réaction d'un dérivé d'acide aminé à thiol protégé de formule (I) ou formule (II) tel que défini dans la revendication 1 avec le groupe amino terminal du polymère hydrophile,
de façon à former un polymère qui comporte un groupe amino primaire ou secondaire terminal.

4. Particule de type noyau-enveloppe comprenant :
une pluralité de polymères séquencés, et
au moins une molécule de charge, par exemple un agent pharmaceutiquement actif ou un polynucléotide, encapsulée dans celle-ci ;
dans laquelle chacun des copolymères séquencés comprend un bloc réactif avec thiol tel que défini dans la revendication 2 et un bloc hydrophile qui est un polymère linéaire de 10 à 1000 motifs monomères ; les blocs hydrophiles des copolymères séquencés sont situés dans la région d'enveloppe de la particule, et les blocs réactifs avec thiol des copolymères séquencés ainsi que la ou les molécule (s) de charge sont situés dans la région de noyau de la particule.

5. Procédé de stabilisation de particule de type noyau-enveloppe selon la revendication 4, le procédé comprenant la mise en contact de la particule de type noyau-enveloppe avec un agent de réticulation comprenant deux ou plus de deux groupes thiol.

6. Composition comprenant la particule de type noyau-enveloppe selon la revendication 4 et un véhicule pharmaceutiquement acceptable.

7. Dérivé d'acide aminé à thiol protégé de formule (I) ou formule (II) 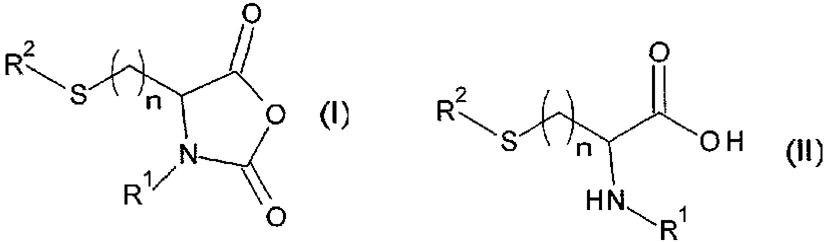 dans lequel
n est 1 ou 2 ;
R¹ est H, alkyle en C₁-C₄, alcényle en C₂-C₄ ou alcynyle en C₂-C₄ ; et
R² est un groupe de formule (A) ou formule (B) 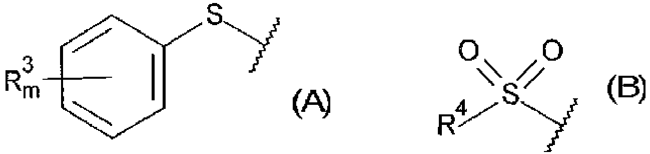 dans lequel
m est 1, 2, 3, 4 ou 5 ;
chaque R³ est indépendamment choisi parmi halogène, cyano et alcanoyle en C₁-C₄ ; et R⁴ est choisi parmi R^{a}-(alkyle en C₁-C₁₆), éthyle, n-propyle, isopropyle, n-butyle, 1-méthylpropyle (sec-butyle), 2-méthylpropyle (isobutyle) et 1,1-diméthyléthyle (tert-butyle), n-pentyle, 1-méthylbutyle, 2-méthylbutyle, 3-méthylbutyle (isopentyle), 2,2-diméthylpropyle, 1-éthylpropyle, 1,1-diméthylpropyle, 1,2-diméthylpropyle, n-hexyle, 1-méthylpentyle, 2-méthylpentyle, 3-méthylpentyle, 4-méthylpentyle (isohexyle), 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle, 1-éthyl-2-méthylpropyle, n-heptyle, 1-méthylhexyle, 2-méthylhexyle, 3-méthylhexyle, 4-méthylhexyle, 5-méthylhexyle, 1-éthylpentyle, 2-éthylpentyle, 3-éthylpentyle, n-octyle, 2-méthylheptyle, 1-éthylhexyle, 2-éthylhexyle, 1,2-diméthylhexyle, 1-propylpentyle, 2-propylpentyle, alcényle en C₂-C₁₆ et alcynyle en C₂-C₁₆, où les alkyle en C₁-C₁₆, éthyle, n-propyle, isopropyle, n-butyle, 1-méthylpropyl (sec-butyle), 2-méthylpropyle (isobutyle) et 1,1-diméthyléthyle (tert-butyle), n-pentyle, 1-méthylbutyle, 2-méthylbutyle, 3-méthylbutyle (isopentyle), 2,2-diméthylpropyle, 1-éthylpropyle, 1,1-diméthylpropyle, 1,2-diméthylpropyle, n-hexyle, 1-méthylpentyle, 2-méthylpentyle, 3-méthylpentyle, 4-méthylpentyle (isohexyle), 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,1,2-triméthylpropyle, 1,2,2-triméthylpropyle, 1-éthyl-1-méthylpropyle, 1-éthyl-2-méthylpropyle, n-heptyle, 1-méthylhexyle, 2-méthylhexyle, 3-méthylhexyle, 4-méthylhexyle, 5-méthylhexyle, 1-éthyipentyle, 2-éthylpentyle, 3-éthylpentyle, n-octyle, 2-méthylheptyle, 1-éthylhexyle, 2-éthylhexyle, 1,2-diméthylhexyle, 1-propylpentyle, 2-propylpentyle, alcényle en C₂-C₁₆ ou alcynyle en C₂-C₁₆ sont non substitués ou comportent 1, 2 ou 3 substituants R^{b} ;
R^{b} est indépendamment choisi parmi halogéno, cyano, nitro, hydroxyle et thiol ;
R^{a} est choisi parmi :
(i) phényle,
(ii) des radicaux monocycliques hétéroaromatiques de 5 ou 6 chaînons ayant 1, 2, 3 ou 4 hétéroatomes en tant que chaînons de cycle qui sont indépendamment choisis parmi 0, S et N,
(iii) des radicaux bicarbocycliques hétéroaromatiques de 8 à 10 chaînons ayant 1, 2, 3 ou 4 hétéroatomes en tant que chaînons de cycle qui sont indépendamment choisis parmi 0, S et N, et
(iv) des radicaux bicarbocycliques aromatiques de 8 à 10 chaînons,
lesdits radicaux (i) à (iv) étant non substitués ou comportant 1, 2, 3 ou 4 substituants R^{c} ; et
R^{c} est indépendamment choisi parmi halogéno, cyano, nitro, hydroxyle, alkyle en C₁-C₄, alcoxy en C₁-C₄, alcényle en C₂-C₄, alcynyle en C₂-C₄ et alcanoyle en C₁-C₄.

8. Dérivé d'acide aminé de la revendication 7, dans lequel R² est un groupe de formule (A) 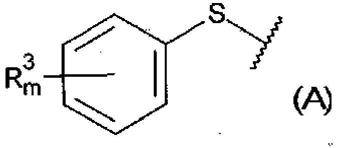 dans lequel m et R³ sont tels que définis dans la revendication 7.

9. Dérivé d'acide aminé de la revendication 8, dans lequel chaque R³ est indépendamment choisi parmi fluoro, chloro et bromo.

10. Dérivé d'acide aminé de la revendication 8, dans lequel
m est 5, et R³ est chloro ; ou
m est 1, 2 ou 3, et R³ est fluoro.

11. Dérivé d'acide aminé de la revendication 7, dans lequel R² est un groupe de formule (B) 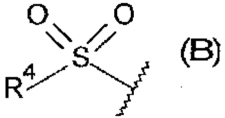 dans lequel R⁴ est tel que défini dans la revendication 7.

12. Dérivé d'acide aminé de la revendication 11, dans lequel R⁴ est choisi parmi éthyle, n-propyle, isopropyle, n-butyle, 1-méthyipropyle (sec-butyle), 2-méthylpropyle (isobutyle) et 1,1-diméthyléthyle (tert-butyle), n-pentyle, 1-méthylbutyle, 2-méthylbutyle, 3-méthylbutyle (isopentyle), 2,2-diméthylpropyle, 1-éthylpropyle, 1,1-diméthylpropyle, 1,2-diméthylpropyle, n-hexyle, 1-méthylpentyle, 2-méthylpentyle, 3-méthylpentyle, 4-méthylpentyle (isohexyle), 1,1-diméthylbutyle, 1,2-diméthylbutyle, 1,3-diméthylbutyle, 2,2-diméthylbutyle, 2,3-diméthylbutyle, 3,3-diméthyibutyle, 1-éthylbutyle, 2-éthylbutyle, 1,1,2-triméthylpropyle, 1,2,2-triméthylpropyle, 1-éthyl-1-méthylpropyle, 1-éthyl-2-méthylpropyle, n-heptyle, 1-méthylhexyle, 2-méthylhexyle, 3-méthylhexyle, 4-méthylhexyle, 5-méthylhexyle, 1-éthylpentyle, 2-éthylpentyle, 3-éthylpentyle, n-octyle, 2-méthylheptyle, 1-éthylhexyle, 2-éthylhexyle, 1,2-diméthylhexyle, 1-propylpentyle, 2- propylpentyle et R^{a}-(alkyle en C₁-C₁₆), où R^{a} est tel que défini dans la revendication 7.

13. Dérivé d'acide aminé de la revendication 11, dans lequel R⁴ est choisi parmi éthyle, butyle, hexyle et benzyle, dans lequel le benzyle est non substitué ou comporte 1, 2, 3 ou 4 substituants R^{c}, et R^{c} est tel que défini dans la revendication 7.

14. Dérivé d'acide aminé de la revendication 11, dans lequel R⁴ est choisi parmi alcényle en C₂-C₁₆ non substitué et alcényle en C₂-C₁₆ partiellement ou complètement halogéné.
